# EUROPEAN PATENT APPLICATION

(11) **EP 4 119 167 A1**
(43) Date of publication of application: **18.01.2023**
(21) Application number: 21768356.4
(22) Date of filing: 10.03.2021
(51) Int. Cl.: A61K 48/00, A61P 1/16, A61P 3/04, A61P 3/06, A61P 3/10, A61P 5/14, A61P 7/04, A61P 9/10, A61P 11/00, A61P 13/12, A61P 19/00, A61P 19/02, A61P 21/04, A61P 25/00, A61P 25/16, A61P 25/28, A61P 27/02, A61P 29/00, A61P 35/00, A61P 37/06

(54) **SINGLE-STRANDED NUCLEIC ACID MOLECULE FOR INDUCING -1 FRAME SHIFT AND COMPOSITION**

(30) Priority: 11.03.2020 JP 2020041637
(71) Applicant: BioComber Co. Ltd, Yokosuka-shi, Kanagawa, 238-0013 (JP)
(72) Inventor: YANO, Takamitsu, Yuzawa-shi Akita 012-0823 (JP)
(74) Representative: Schulz Junghans Patentanwälte PartGmbB
(86) International application number: PCT/JP2021/009545
(87) International publication number: WO 2021/182517

(57) **Abstract**

It is an objective of the present invention to provide a substance that is safe for a living individual and allows to express of a protein with an amino acid sequence more similar to that of an original protein even from a gene causing out-of-frame in a state in which deletion of protein length is suppressed, and a composition containing the substance. The objective is achieved by a single-stranded nucleic acid molecule containing in the direction from the 5' end side to the 3' end side a first sequence complementary to a target sequence of a gene of interest and a second sequence forming a stem-loop structure; and a composition containing the single-stranded nucleic acid molecule as an active ingredient and the like.

## Description

### Technical Field

The present invention relates to a single-stranded nucleic acid molecule for inducing -1 frameshift, and a composition containing the single-stranded nucleic acid molecule.

### Background Art

Genetic diseases are caused by impaired expression of a gene, for example, by reducing or increasing the expression level of a gene caused by any gene aberration. Known examples of the genetic diseases include muscular dystrophy, cancer, multiple sclerosis, spinal muscle atrophy, Rett syndrome, amyotrophic lateral sclerosis, fragile X syndrome, Prader-Willi syndrome, xeroderma pigmentosum, porphyria, Werner syndrome, fibrodysplasia ossificans progressiva, infantile neuronal ceroid lipofuscinosis, Alzheimer's disease, Tay-Sachs disease, degenerative neurological disorder, Parkinson's disease, rheumatoid arthritis, graft-versus-host disease, arthritis, hematopoietic disease, von Willebrand disease, ataxia-telangiectasia, thalassemia, kidney stone, osteogenesis imperfecta, liver cirrhosis, neurofibromatosis, bullous disease, lysosomal storage disease, Hurler syndrome, cerebellar ataxia, tuberous sclerosis complex, familiar polycythemia, immunodeficiency, kidney disease, lung disease, cystic fibrosis, familial hypercholesterolemia, pigmentary retinopathy, amyloidosis, atherosclerosis, giantism, dwarfism, hypothyroidism, hyperthyroidism, aging, obesity, diabetes, Niemann-Pick disease, and Marfan syndrome.

Among the genetic diseases, nonsense mutation type genetic diseases are caused by inhibition of protein translation due to premature termination codon (PTC) which is formed through point mutation on a gene.

When PTC is formed as a termination codon in the upstream region (the 5' end side) from the position of an original termination codon on mRNA obtained by gene transcription, a protein obtained through translation may become shorter, and thus may not be functional. In addition, when PTC is formed through incorrect translation from mRNA, nonsense-mediated mRNA decay (NMD), which is an intracellular mRNA surveillance mechanism, may be activated. As a result, mRNA with PTC may be aggressively degraded in a cell to reduce or disappear the protein production. Such impaired gene expression leads to genetic diseases.

PTC can be also caused by a frameshift mutation. PTC is formed by the shift of the reading frame (frameshift) during translation when nucleotides with a number other than multiple of three in a gene are deleted or inserted.

Therefore, once PTC is formed due to out-of-frame mutation such as nonsense mutation and frameshift mutation, no functional protein can be obtained. On the other hand, the in-frame mutation is a mutation in which the reading frame is kept intact. A protein may be produced regardless of in-frame mutation although the produced protein may have a different portion from an original protein. Therefore, in general, genetic diseases caused by out-of-frame mutation tend to be more serious than those caused by in-frame mutation.

Duchenne muscular dystrophy (DMD), which is one of the genetic diseases caused by out-of-frame mutation, is caused by a mutation of the dystrophin gene on the X chromosome. For example, if part of exons of the dystrophin gene on the X chromosome lacks, PTC can be formed on mRNA, leading to the problem that translation can be interrupted or terminated. As a result, normal expressions of functional dystrophin and dystrophin-related proteins cannot be achieved. This causes a deficiency of functional dystrophin proteins in muscle tissues, resulting in the development of DMD.

As methods for treating genetic diseases caused by out-of-frame mutation such as DMD, chemotherapies including administrating substances such as a substance causing exon skipping and a substance having read-through activity have been expected.

Exon skipping is a technique to skip one or more exons with out-of-frame mutation so that out-of-frame mutation can be converted to in-frame mutation, and a protein encoded by mRNA lacking the exons can be obtained. The protein obtained by exon skipping is a truncated protein with shorter amino acids in length than those of an original protein. As substances causing exon skipping, for example, the compound and antisense nucleic acid described in Patent Documents 1 and 2 (the entire disclosures of which are incorporated herein by reference) below are known.

On the other hand, read-through activity means an activity that affects the ribosome and allows the ribosome to make progress with translation while skipping reading PTC formed by out-of-frame mutation. As a result of read-through activity, a functional protein can be produced. Examples of substances having read-through activity include the compound described in Patent Document 3 (the entire disclosure of which is incorporated herein by reference) below.

Non-Patent Document 1 (the entire disclosure of which is incorporated herein by reference) below discloses a method for identifying a frameshift gene in a genome and circular code information used in the method.

### Citation List

### Patent Documents

[Patent Document 1] Japanese Patent No. 5950818
[Patent Document 2] Japanese Patent No. 5363655
[Patent Document 3] Japanese Patent No. 5705136

### Non-Patent Document

[Non-Patent Document 1] J Comput Sci Syst Biol, 2011, Vol. 4(1), pp. 7-15

### Summary of the Invention

### Problems to be Solved by the Invention

Exon skipping with the compound and/or antisense nucleic acid of Patent Documents 1 and 2 may be used to skip reading mutated exons to read genetic information up to the end while not causing PTC. However, just skipping in reading only the mutated exons cannot correct impaired reading of genetic information. Therefore, the problem is that exon skipping needs to skip not only the mutated exons but also one or more exons around the mutated exons. Furthermore, there is a problem that a protein produced is shortened depending on the number of exons skipped and thus no functional protein can be obtained.

The substance having a read-through activity like the compound of Patent Document 3 may be used to skip reading PTC. However, the problem is that since the substance having read-through activity cannot also correct impaired reading, a protein produced is different in amino acid composition from an original protein. In addition, read-through can be mainly achieved depending on probability theory. As a result, the problem is that since read-through can occur for other amino acids instead of PTC, continuous protein synthesis tends to be not achieved. Furthermore, the compound of Patent Document 3, arbekacin, is an antibiotic substance and thus has high toxicity to a living individual.

Besides, there are few reports on methods for translating a protein having an amino acid sequence more similar to that of an original protein from a gene causing out-of-frame mutation in a state in which a lack of a protein length is suppressed as much as possible.

Accordingly, it is an objective of the present invention to provide a substance that is safe for a living individual and allows to translate of a protein with an amino acid sequence more similar to that of an original protein even from a gene causing out-of-frame mutation in a state in which a lack of a protein length is suppressed, when compared with a substance causing exon skipping or having read-through activity, and to provide a composition containing the substance.

### Means for Solving the Problems

To solve the problems, the present inventor conducted extensive research on correcting impaired reading of genetic information without skipping exons. As a result, the present inventor reached an assumption that if the reading frame can be shifted by one nucleotide in the 5' direction or one nucleotide in the 3' direction, or two nucleotides in the 3' direction at or around a mutated portion, it is possible to translate a protein with an amino acid sequence more similar to that of an original protein while retaining the length of protein produced as much as possible.

Based on the above assumption, as a result of repeated trial and error, the present inventor surprisingly succeeded in correcting impaired reading of genetic information without skipping reading exons, by using a single-stranded nucleic acid molecule containing a sequence complementary to a target sequence of a gene of interest and a sequence forming a predetermined stem-loop structure and inducing -1 frameshift at or around a mutated portion to shift reading frame. In addition, the single-stranded nucleic acid molecule can be decomposed by a nucleolytic enzyme in a living individual and thus is safer for a living individual than synthetic compounds.

Finally, the present inventor succeeded in creating the single-stranded nucleic acid molecule and a composition containing the single-stranded nucleic acid molecule as an active ingredient. The present invention has been completed based on such successful examples and findings.

Therefore, according to aspects of the present invention, the following single-stranded nucleic acid molecule and composition are provided:
[1] A single-stranded nucleic acid molecule, containing in the direction from 5' end side toward 3' end side a first sequence complementary to a target sequence of a gene of interest, and a second sequence forming a stem-loop structure.
[2] The single-stranded nucleic acid molecule according to claim 1, wherein the stem-loop structure has a stem portion the ends of which are composed of cytosine and guanine.
[3] The single-stranded nucleic acid molecule according to any one of [1] or [2], wherein the stem-loop structure has a loop portion that is primarily composed of cytosine.
[4] The single-stranded nucleic acid molecule according to any one of [1] to [3], wherein the stem-loop structure has a stem portion composed of 4 base pairs to 20 base pairs.
[5] The single-stranded nucleic acid molecule according to any one of [1] to [4], the second sequence is represented in SEQ ID NO: 17 or SEQ ID NO:18.
[6] The single-stranded nucleic acid molecule according to any one of [1] to [5], wherein the first sequence has 8 nucleotides to 16 nucleotides in length.
[7] The single-stranded nucleic acid molecule according to any one of [1] to [6], wherein the gene has two or more consecutive identical nucleotides in 9 nucleotides to 15 nucleotides located upstream of the target sequence.
[8] The single-stranded nucleic acid molecule according to any one of [1] to [6], wherein the gene has two or more sets of a portion composed of two or more consecutive identical nucleotides in 9 nucleotides to 15 nucleotides located upstream of the target sequence.
[9] A composition for inducing a -1 frameshift, containing the single-stranded nucleic acid molecule according to any one of [1] to [8] as an active ingredient.
[10] The composition according to [9], wherein the composition is a pharmaceutical composition for preventing and/or treating a genetic disease caused by impaired expression of a gene, by inducing a -1 frameshift to normalize the expression of the gene.
[11] The composition according to [10], wherein the genetic disease is selected from a group consisting of muscular dystrophy, cancer, multiple sclerosis, spinal muscle atrophy, Rett syndrome, amyotrophic lateral sclerosis, fragile X syndrome, Prader-Willi syndrome, xeroderma pigmentosum, porphyria, Werner syndrome, fibrodysplasia ossificans progressiva, infantile neuronal ceroid lipofuscinosis, Alzheimer's disease, Tay-Sachs disease, degenerative neurological disorder, Parkinson's disease, rheumatoid arthritis, graft-versus-host disease, arthritis, hematopoietic disease, von Willebrand disease, ataxia-telangiectasia, thalassemia, kidney stone, osteogenesis imperfecta, liver cirrhosis, neurofibromatosis, bullous disease, lysosomal storage disease, Hurler syndrome, cerebellar ataxia, tuberous sclerosis complex, familiar polycythemia, immunodeficiency, kidney disease, lung disease, cystic fibrosis, familial hypercholesterolemia, pigmentary retinopathy, amyloidosis, atherosclerosis, giantism, dwarfism, hypothyroidism, hyperthyroidism, aging, obesity, diabetes, Niemann-Pick disease, and Marfan syndrome.
[12] A method for treating and/or preventing a genetic disease, containing administrating an effective amount of the single-stranded nucleic acid molecule according to any one of [1] to [8] or the composition according to [9], to a living individual developing or being at risk of developing the genetic disease caused by impaired expression of a gene of interest.
[13] A method for ameliorating impaired expression of a gene, containing administrating an effective amount of the single-stranded nucleic acid molecule according to any one of [1] to [8] or the composition according to [9], to a cell, a tissue, an organ or a living individual in which the impaired expression of the gene of interest is observed.
[14] The method according to any one of [12] or [13], the genetic disease is selected from a group consisting of muscular dystrophy, cancer, multiple sclerosis, spinal muscle atrophy, Rett syndrome, amyotrophic lateral sclerosis, fragile X syndrome, Prader-Willi syndrome, xeroderma pigmentosum, porphyria, Werner syndrome, fibrodysplasia ossificans progressiva, infantile neuronal ceroid lipofuscinosis, Alzheimer's disease, Tay-Sachs disease, degenerative neurological disorder, Parkinson's disease, rheumatoid arthritis, graft-versus-host disease, arthritis, hematopoietic disease, von Willebrand disease, ataxia-telangiectasia, thalassemia, kidney stone, osteogenesis imperfecta, liver cirrhosis, neurofibromatosis, bullous disease, lysosomal storage disease, Hurler syndrome, cerebellar ataxia, tuberous sclerosis complex, familiar polycythemia, immunodeficiency, kidney disease, lung disease, cystic fibrosis, familial hypercholesterolemia, pigmentary retinopathy, amyloidosis, atherosclerosis, giantism, dwarfism, hypothyroidism, hyperthyroidism, aging, obesity, diabetes, Niemann-Pick disease, and Marfan syndrome.

### Effect of the Invention

According to the present invention, -1 frameshift can be induced to produce a protein with an amino acid sequence more similar to that of an original protein holding its entire length longer from a gene having an out-of-frame mutation. Therefore, according to the present invention, a functional protein from a mutated gene is expected to be produced.

Therefore, according to the present invention, recovering the impaired expression of a gene of interest is expected to treat and/or prevent genetic diseases caused by impaired expression of a gene such as reduced or increased expression level of a gene. Such genetic diseases include muscular dystrophy, cancer, multiple sclerosis, spinal muscle atrophy, Rett syndrome, amyotrophic lateral sclerosis, fragile X syndrome, Prader-Willi syndrome, xeroderma pigmentosum, porphyria, Werner syndrome, fibrodysplasia ossificans progressiva, infantile neuronal ceroid lipofuscinosis, Alzheimer's disease, Tay-Sachs disease, degenerative neurological disorder, Parkinson's disease, rheumatoid arthritis, graft-versus-host disease , arthritis , hematopoietic disease, von Willebrand disease, ataxia-telangiectasia, thalassemia, kidney stone, osteogenesis imperfecta, liver cirrhosis, neurofibromatosis, bullous disease, lysosomal storage disease, Hurler syndrome, cerebellar ataxia, tuberous sclerosis complex, familiar polycythemia, immunodeficiency, kidney disease, lung disease, cystic fibrosis, familial hypercholesterolemia, pigmentary retinopathy, amyloidosis, atherosclerosis, giantism, dwarfism, hypothyroidism, hyperthyroidism, aging, obesity, diabetes, Niemann-Pick disease, and Marfan syndrome.

### Brief Description of Drawings

[Fig.1] Fig.1 shows the coding sequence (CDS) of EGFP-Human α-Tubulin, as described in the Examples below.
[Fig.2] Fig.2 shows the imaging results of the fluorescence microscope of Example 1, as described in the Examples below.
[Fig.3] Fig.3 shows the CDS of the DHFR gene, as described in the Examples below.
[Fig.4A] Fig.4A shows the nucleotide sequence of the reverse primer used in Example 2, as described in the Examples below.
[Fig.4B] Fig.4B shows the amino acid sequence of DHFR protein and the amino acid sequence of DHFR-His protein, as described in the Examples below.
[Fig.5A] Fig.5A shows the imaging result of the entire gel after electrophoresis and staining in Example 2, as described in the Examples below.
[Fig.5B] Fig.5B is the enlarged view of Fig. 5A focusing on the portion where His-tagged DHFR protein of TEST 2 Elution 1 and Elution 2 appeared.
[Fig.5C] Fig.5C is the enlarged view of Fig. 5A focusing on the portion where DHFR protein and DHFR protein fragments in NC, PC, and TEST 1 appeared.
[Fig.6A] Fig.6A shows the target sequence Position 1 set around the breakpoint between exon 44 and exon 51 of the mutated dystrophin gene, as described in the Examples below.
[Fig.6C] Fig.6C shows the target sequence Position 2 set around the breakpoint between exon 44 and exon 51 of the mutated dystrophin gene, as described in the Examples below.

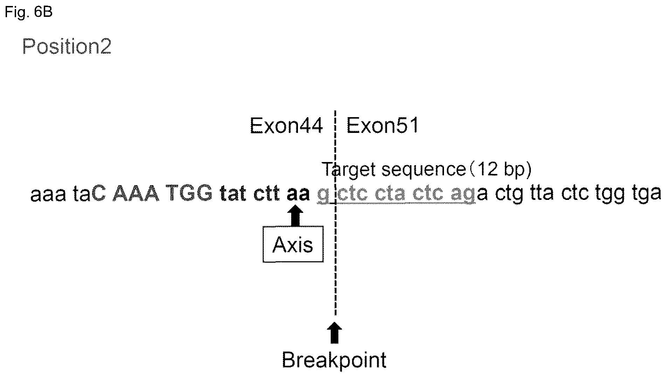

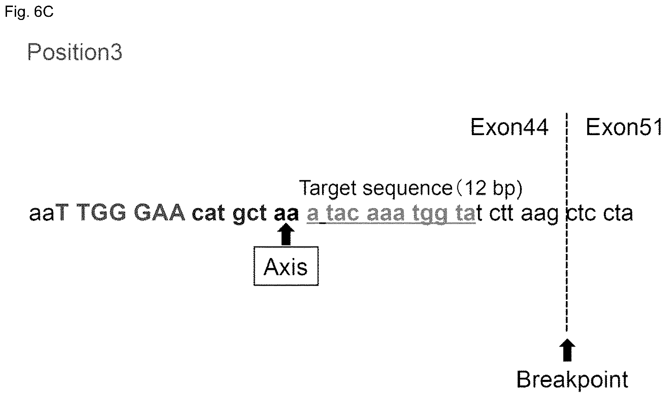

[Fig.6C] Fig.6C shows the target sequence Position 3 set around the breakpoint between exon 44 and exon 51 of the mutated dystrophin gene, as described in the Examples below.

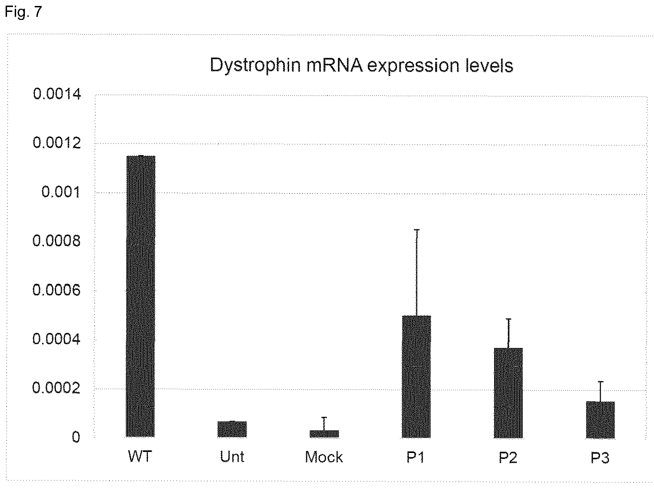

[Fig.7] Fig.7 is the graph showing the results of the relative amount of dystrophin mRNA expression level relative to GAPDH mRNA expression level described in Example 3, as described in the Examples below.

### Modes for Carrying Out the Invention

While each embodiment of the present invention will now be described in further detail, the scope of the present invention is not limited to what is described in this section; rather, the present invention may take various other forms to the extent that its objective is achieved.

Unless otherwise specified, each term used herein is used in the meaning commonly used by those skilled in the art of medicine, biology, and the like, and should not be construed to have any unduly limiting meaning. Also, any speculations and theories herein are made based on the knowledge and experiences of the present inventor and as such, the present invention is not bound by any such speculations and theories.

While the term "composition" is not particularly limited and means composition as well known, it is, for example, comprised of a combination of two or more components.

The term "active ingredient" means a component that characterizes the use of a composition.

The term "and/or" means any one of, or any combination of two or more of, or a combination of all of the listed related items.

The term "content" is equivalent to "concentration" or "amount added" and means the proportion of a component relative to the total amount of a composition containing the component. It should be noted, however, that the total amount of the contents of components does not exceed 100 %. The term "effective amount" means an amount at which an effect of an active ingredient or a composition is exhibited.

The wording "to" for indicating a range of values is intended to include values preceding and following the wording; for example, "0 % to 100 %" means a range from 0 % or more and 100 % or less. The wordings "more than" and "less than" mean the lower limit and the upper limit, respectively, without including the following numerical value. For example, "more than 1" is a numerical value greater than 1, and "less than 100" is a numerical value smaller than 100.

The terms "include", "comprise" and "contain" mean that an element(s) other than an element(s) as explicitly indicated can be added as inclusions, which are, for example, synonymous with "at least include," but encompasses the meaning of "consist of" and "substantially consist of'. In other words, the terms may mean, for example, to include an element(s) as explicitly indicated as well as any one element or any two or more elements, to consist of an element(s) as explicitly indicated, or substantially consist of an element(s) as explicitly indicated. Such elements include limitations such as components, steps, conditions, and parameters.

While the term "stem-loop structure", which is also referred to as "hairpin loop", is not particularly limited and has commonly used meaning, it is, for example, a structure comprised of a first and a second sequences complementary to each other as well as a third sequence between the first and second sequences. In this case, the first and second sequences form base pairs to form a double-stranded portion (stem portion), the third sequence forms no base pairs to form a loop portion, and the whole structure exhibits a stem-loop structure.

The term "complementary base pair" or simply "base pair" means Watson-Crick type base pair, and wobble base pair means the combinations of adenine (A) and thymine (T) or uracil (U), and of guanine (G), and cytosine (C), as well as the combination of guanine (G) and uracil (U). As used herein, adenine, thymine, uracil, guanine, and cytosine, and the corresponding symbols A, T, U, G, and C are referred to as "base" or "nucleotide". As used herein, "base" and "nucleotide" are treated as interchangeable terms.

The term "end of stem portion" refers to nucleotides at one end of two ends of a stem portion that is not bound to nucleotides of a loop portion.

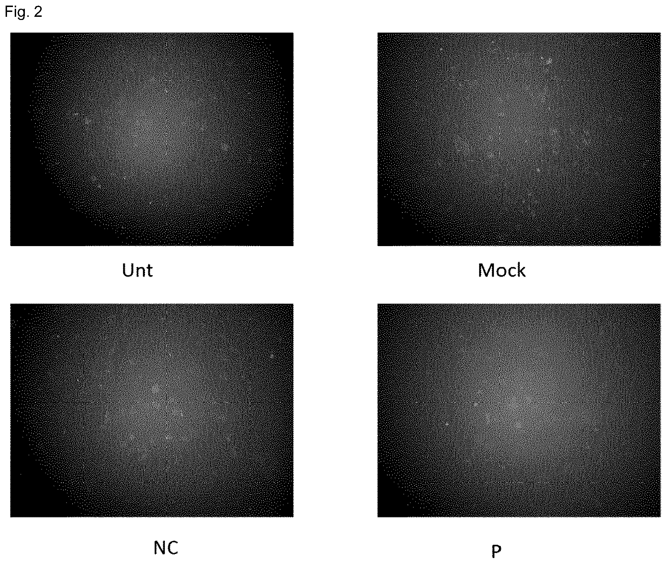

The term "-1 frameshift" means a frameshift that shifts the reading frame of a codon by one nucleotide in the direction toward the 5' end side among frameshifts meaning a shift of the reading frame of the codon. For example, Scheme (I), which is cited in Figure 2 of Non-Patent Document 1, illustrates that the mRNA sequence represented as Scheme (I) is read three nucleotides at a time in order from the start codon, AUG, to CGU, GCU, and GUG by ribosomal RNA (Frame 0). In case the reading frame is shifted by one nucleotide in the direction toward the 5' end side at the frameshift site (hereinafter also referred to as slipping site or slippage site) shown herein, the last G in codon GUG is set as the beginning, and GGC is read, and in turn, GAAand AUA are read (Frame 2). As such, a frameshift to be shifted by one nucleotide in the direction toward the 5' end side at a frameshift site is called "-1 frameshift".

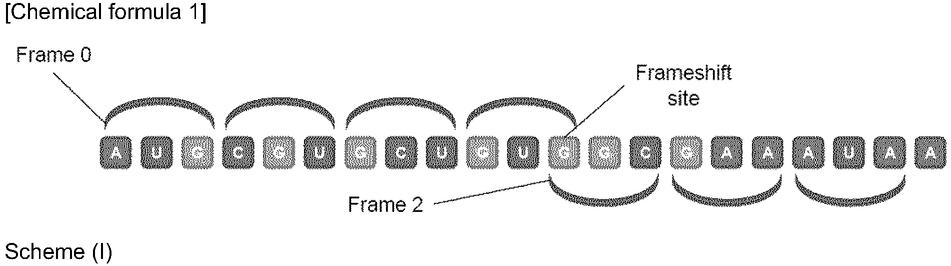

The term "out-of-frame mutation" means a mutation in which the reading frame on the open reading frame (ORF) of mRNA is shifted. If an out-of-frame mutation occurs, the result is that a protein with an amino acid sequence different from that of an original non-mutated protein is produced or a shorter protein than an original non-mutated protein is produced due to premature termination codon (PTC) formed in the 5' end side (upstream) from an original stop codon.

The term "nucleic acid molecule", which is also simply referred to as nucleic acid, is as usually meant and thus means a polymer in which nucleotides composed of ribonucleotides, deoxyribonucleotides, and their analogs are linked to each other by phosphodiester bonds. The term "nucleotide" may be called "base", and the terms "nucleotide sequence" and "base sequence" are synonymous. The term "nucleotide sequence" and "base sequence" may be simply referred to as "sequence of nucleic acid molecule". Typical examples of a nucleic acid molecule include ribonucleic acid (RNA) composed of ribonucleotides as well as deoxyribonucleic acid (DNA) composed of deoxyribonucleotides.

The term "single-stranded nucleic acid molecule" means a single nucleic acid molecule that does not form complementary base pairs with any other nucleic acid molecules. The single-stranded nucleic acid molecule may have a stem portion in which the intramolecular sequences form complementary base pairs to each other.

The term "expression" means the synthesis of mRNA from a gene (transcription), synthesis of a protein from mRNA (translation), or both.

The term "upstream" concerning a sequence means the 5' end side in a nucleotide sequence or the N-terminal side in an amino acid sequence. The term "downstream" concerning a sequence means the 3' end side in a nucleotide sequence or the C-terminal side in an amino acid sequence.

The number of digits of an integer equals its significant figure. For example, 1 has one significant figure and 10 has two significant figures. For a decimal number, the number of digits after a decimal point equals its significant figure. For example, 0.1 has one significant figure and 0.10 has two significant figures.

### [Single-stranded nucleic acid molecule]

The single-stranded nucleic acid molecule according to one embodiment of the present invention contains in the direction from the 5' end side to the 3' end side a first sequence complementary to a target sequence of a gene of interest, and a second sequence forming a stem-loop structure.

The single-stranded nucleic acid molecule can be used to induce -1 frameshift when the mRNA of a gene of interest is translated. In other words, the single-stranded nucleic acid molecule has a -1 frameshift-inducing effect. The mechanism of the -1 frameshift-inducing effect of the single-stranded nucleic acid molecule will be explained hereinafter using a schematic drawing of mRNA of a gene of interest represented as Scheme (II).

[Chemical formula 2]

5'-N₃₇N₃₆N₃₅N₃₄N₃₃N₃₂N₃₁-N₂₆N₂₅N₂₄N₂₃N₂₂N₂₁-N₁₂N11-Target sequence -3' Scheme (II)

The first sequence in the single-stranded nucleic acid molecule is bound to a target sequence in mRNA of a gene of interest by forming complementary base pairs. When mRNA of the gene of interest is translated in a state with the single-stranded nucleic acid molecule bound, a -1 frameshift occurs to shift the reading frame by one nucleotide forward in front of 14 nucleotides upstream of the target sequence. Scheme (II) illustrates that the sense codon N₃₆N₃₅N₃₄, which starts from N₃₆ located in 14 nucleotides upstream of the target sequence, is shifted by one nucleotide forward by -1 frameshift to become the sense codon N₃₇N₃₆N₃₅. In this case, it is considered that -1 frameshift occurs after adding the amino acids corresponding to the first sense codon N36N35N34 and the subsequent second sense codon N33N32N31 to a protein. As a result, the ribosome may be slipped toward the position of the sense codons N37N36N35 and N34N33N32. This leads to a presumption that the amino acid sequence of the protein synthesized would have an amino acid encoded by the first sense codon N₃₆N₃₅N₃₄, an amino acid encoded by the second sense codon N33N32N31, an amino acid encoded by the sense codon N₃₁N₂₆N₂₅, and an amino acid encoded by the sense codon N₂₄N₂₃N₂₂. The term "nucleotide (base) upstream of a sequence" from a nucleotide means, as the basis of the 5' end of the sequence, a nucleotide (base) which is located in the 5' end side closer than the latter nucleotide. The term "nucleotide (base) downstream of a sequence" from a nucleotide means, as the basis of the 3' end of the sequence, a nucleotide (base) which is located in the 3' end side closer than the latter nucleotide.

The target sequence can be optionally determined depending on a site intended to achieve -1 frameshift. The target sequence can be set at a position starting from 14 nucleotides downstream of a site intended to achieve a -1 frameshift (N₃₆ in Scheme (II)). The sequence of 9 nucleotides upstream to 15 nucleotides upstream of the target sequence (N37N36N35N34N33N32N31 in Scheme (II)) has preferably two or more consecutive identical nucleotides such as CC, AA, UU, and GG. Furthermore, the sequence of 9 nucleotides upstream to 15 nucleotides upstream of the target sequence has preferably two or more sets of a portion comprised of two or more consecutive identical nucleotides. The number of nucleotides in the above expression "9 nucleotides upstream to 15 nucleotides upstream of the target sequence" is a number in a case wherein the sequence N26N25N24N23N22N21 in Scheme (II) (referred to as spacer sequence) is 6 nucleotides. The spacer sequence may be composed of 3 nucleotides to 8 nucleotides. For example, when the spacer sequence is composed of 8 nucleotides, the above expression "9 nucleotides upstream to 15 nucleotides upstream of the target sequence" is replaced with the expression "11 nucleotides upstream to 17 nucleotides upstream of the target sequence".

The first sequence contains a sequence complementary to the target sequence. So long as the length of the first sequence is sufficient to bind to the target sequence, it is not particularly limited. Examples of the length include 5 nucleotides to 25 nucleotides, and given the binding capability to the target sequence, preferably 6 nucleotides to 20 nucleotides, more preferably 8 nucleotides to 16 nucleotides, and still more preferably 11 nucleotides to 13 nucleotides.

The first sequence may be either completely complementary or partially complementary to the target sequence. Given the binding capability to the target sequence, the first sequence is preferably completely complementary to the target sequence.

The first sequence can be determined based on the nucleotide sequence of the target sequence.

The second sequence forms a stem-loop structure. When the single-stranded nucleic acid molecule binds to the mRNA of the gene of interest by forming complementary base pairs between the target sequence and the first sequence, the end of the stem portion of the stem-loop structure retains the intramolecular double-stranded structure and does not form any complementary base pairs with mRNA of the gene of interest. This will now be explained by illustrating, but not limited to, Scheme (III) representing the single-stranded nucleic acid molecule of one embodiment of the present invention.

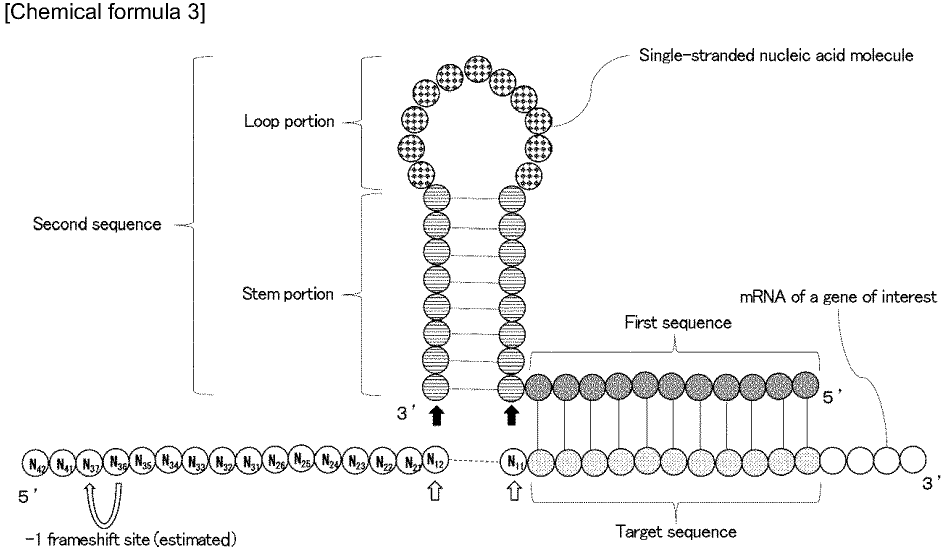

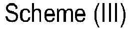

In Scheme (III), "∘" (white circle) represents one nucleotide. The solid line represents a relationship of complementary base pair (hydrogen bond), and the dotted line represents a bond between adjacent nucleotides (phosphodiester bond). As shown in Scheme (III), two nucleotides at the end of the stem portion as indicated by the black arrow do not form a complementary base pair with the opposing mRNA sequence (N₁₂, N₁₁) of the gene of interest as indicated by the white arrow. As such, in the second sequence, the end of the stem-loop structure retains the intramolecular double-stranded structure as a stem portion.

In the stem-loop structure of the second sequence, the length of the stem portion is sufficient to form the intramolecular double-stranded structure and is not particularly limited. Examples of the length include the length forming 3 base pairs to 50 base pairs and given stably retaining the intramolecular double-stranded structure, preferably the length forming 4 base pairs to 20 base pairs, more preferably the length forming 5 base pairs to 10 base pairs, and still more preferably the length forming about 8 base pairs.

While the stem portion may be either a complete intramolecular double-stranded structure or a double-stranded structure partly having one or more single-stranded structures in the middle, the stem portion is preferably a complete intramolecular double-stranded structure given stably retaining the intramolecular double-stranded structure. Even if the double-stranded structure partly has one or more single-stranded structures in the middle, two nucleotides at the end of the stem portion preferably from a complementary base pair between them.

The nucleotide sequence of the stem portion is not particularly limited so long as it is a sequence to form the intramolecular double-stranded structure. Two nucleotides at the end of the stem portion are preferably a combination of cytosine and guanine or a combination of adenine and uracil, and more preferably a combination of cytosine and guanine. One embodiment of one nucleotide sequence constituting the stem portion is, but is not limited to, a sequence represented by SEQ ID NO: 15 (5'-CCGCAUUA-3'). A sequence that forms complementary base pairs to form a complete intramolecular double-stranded structure with the sequence represented by SEQ ID NO: 15 is represented by SEQ ID NO: 16 (5'-UAGUGUGG-3').

In the stem-loop structure of the second sequence, the length of the loop portion is sufficient to retain the single-stranded structure and is not particularly limited. Examples of the length include from 3 nucleotides to 25 nucleotides and given stably retaining the single-stranded structure, preferably from 5 nucleotides to 20 nucleotides, more preferably from 8 nucleotides to 15 nucleotides, and still more preferably about 11 nucleotides.

While the loop portion may be either a complete single-stranded structure or a single-stranded structure partially having one or more double-stranded structures in the middle, the loop portion is preferably a complete single-stranded structure given stably retaining the single-stranded structure.

The nucleotide sequence of the loop portion is not particularly limited so long as it is a sequence that retains a single-stranded structure. Examples of the nucleotide sequence include a sequence that does not form an intramolecular double-stranded structure, preferably a sequence composed of cytosine and adenine or uridine, a sequence composed of guanine and adenine, a sequence composed of cytosine, guanine, adenine or uridine, more preferably a sequence mainly composed of cytosine, which has cytosine in the ratio equal to or more than 80%, and still more preferably a sequence completely composed of cytosine. One embodiment of the nucleotide sequence of the loop portion is but is not limited to, a sequence composed of eleven cytosines.

The length of the second sequence is the sum of the lengths of the stem portion and the loop portion. Examples of the length include 9 nucleotides to 65 nucleotides and given stably retaining the stem-loop structure, preferably 15 nucleotides to 50 nucleotides, more preferably 18 nucleotides to 35 nucleotides, and still more preferably about 27 nucleotides.

The length of the loop portion in the second sequence may be longer than, shorter than, or the same as the length of the stem portion. Given the efficiency of -1 frameshift induction, the length of the loop portion is preferably longer than or the same as the length of the stem portion, more preferably longer than the length of the stem portion, still more preferably 1.2 times to 8 times longer than the stem portion, and even still more preferably 1.3 times to 1.5 times longer than the length of the stem portion.

While the second sequence forms a stem-loop structure composed of the stem portion and the loop portion, examples of the second sequence include a sequence represented by SEQ ID NO: 17 and a sequence represented by SEQ ID NO: 18.

The single-stranded nucleic acid molecule contains the first sequence and the second sequence in the direction from the 5' end side to the 3' end side. While the single-stranded nucleic acid molecule may have 1, 2, 3, 4, 5, 6, 7, 8, or 9 nucleotides added at the 5' end side of the first sequence, at the 3' end side of the second sequence and/or between the first sequence and the second sequence, it is preferably composed of the first sequence and the second sequence.

The length of the single-stranded nucleic acid molecule composed of the first sequence and the second sequence is the sum of the lengths of the first sequence and the second sequence. Examples of the length include 14 nucleotides to 90 nucleotides, preferably 21 nucleotides to 70 nucleotides, more preferably 26 nucleotides to 51 nucleotides, and still more preferably 38 nucleotides to 40 nucleotides. While the length of the second sequence in the single-stranded nucleic acid molecule is preferably longer than the length of the first sequence, the length of the first sequence is preferably longer than the length of the stem portion of the second sequence.

Each nucleotide contained in the single-stranded nucleic acid molecule may be a natural type nucleotide (natural nucleotide) or modified type nucleotide (modified nucleotide). Furthermore, the single-stranded nucleic acid molecule may be composed of any one of natural nucleotides only, modified nucleotides only, and a combination of natural nucleotides and modified nucleotides.

While the modified nucleotide is not particularly limited, examples of the modified nucleotide include nucleotides with sugar moiety modified, such as a nucleotide with 2'-O-alkylated D-ribofuranose and a nucleotide with 2-O, 4'-C-alkylated D-ribofuranose; nucleotides with phosphodiester bond moiety modified, such as thiolated nucleotide; nucleotides with base moiety modified; and nucleotides modified by a combination thereof. Since the single-stranded nucleic acid molecule with modified nucleotides can have advantages including a strong binding capability to RNA and high resistance against nuclease, it can be expected that the above molecule has a more improved -1 frameshift-inducing effect than the single-stranded nucleic acid molecules composed of natural nucleotides only. The modified nucleotide may be also peptide nucleic acid (PNA).

Examples of sugar modifications of the modified nucleotides include 2'-O-alkylation of D-ribofuranose (e.g., 2'-O-methylation, 2'-O-aminoethylation, 2'-O-propylation, 2'-O-allylation, 2'-O-methoxyethylation, 2'-O-butylation, 2'-O-pentylation, 2'-O-propargylation), 2'-O, 4'-C-alkyleneation of D-ribofuranose (e.g., 2'-O, 4'-C-ethyleneation, 2'-O, 4'-C-methyleneation, 2'-O, 4'-C-propyleneation, 2'-O, 4'-C-tetramethyleneation, 2'-O, 4'-C-pentamethyleneation), 3'-deoxy-3'-amino-2'-deoxy-D-ribofuranose, and 3'-deoxy-3'-amino-2'-deoxy-2'-fluoro-D-ribofuranose.

Examples of phosphate diester bond modifications of the modified nucleotides include phosphorothioate bond, methylphosphonate bond, methylthiophosphonate bond, phosphorodithioate bond, and phosphoramidite bond.

Examples of nucleotide base modifications of the modified nucleotides include 5-methylation, 5-fluorolation, 5-bromolation, 5-iodolation, N4-methylation, of cytosine; 5-demethylation (uracil), 5-fluorolation, 5-bromolation, 5-iodolation, of thymidine; N6-methylation, 8-bromolation, of adenine; and N2-methylation, 8-bromolation, of guanine.

The single-stranded nucleic acid molecule may be in the form of a salt, i.e., a pharmaceutically acceptable salt of the single-stranded nucleic acid molecule. Examples of the salt include, but are not limited to, alkali metal salts such as sodium salt, potassium salt and lithium salt; alkaline earth metal salts such as calcium salt and magnesium salt; metal salts such as aluminum salt, iron salt, zinc salt, copper salt, nickel salt and cobalt salt; inorganic salts such as ammonium salt; organic salts such as t-octylamine salt, dibenzylamine salt, morpholine salt, glucosamine salt, phenylglycine alkyl ester salt, ethylenediamine salt, N-methylglucamine salt, guanidine salt, diethylamine salt, triethylamine salt, dicyclohexylamine salt, N, N'-dibenzylethylenediamine salt, chloroprocine salt, prokine salt, diethanolamine salt, N-benzyl-phenethylamine salt, piperazine salt, tetramethylammonium salt and tris (hydroxymethyl) aminomethane salt; salts of hydrohalogenic acid such as hydrofluoride, hydrochloride, hydrobromide and hydroiodide; inorganic acid salts such as nitrate, perchlorate, sulfate and phosphate; lower alkane sulfonate salts such as methane sulfonate, trifluoromethane sulfonate and ethane sulfonate; aryl sulfonate salts such as benzene sulfonate and p-toluene sulfonate; organic acid salts such as acetate, malate, fumarate, succinate, citrate, tartrate, oxalate and maleate; amino acid salts such as glycine salt, lysine salt, arginine salt, ornithine salt, glutamate salt and asparaginate salt.

The single-stranded nucleic acid molecule may be in the form of a solvate such as a hydrate.

The single-stranded nucleic acid molecule may be in the form of a prodrug. Examples of the prodrug include amides, esters, carbamates, carbonates, ureides, and phosphates.

The single-stranded nucleic acid molecule can be synthesized using known methods and devices. For example, DNA can be synthesized according to the method described in Sinha et al. (N.D. Sinha et al., Nucleic Acids Research, 12, 4539 (1984); the entire disclosure of which is incorporated herein by reference). In addition, RNA can be synthesized by a method including inserting the synthesized DNA into a vector and carrying out transcription reaction using the resulting vector recombinant as a template and RNA polymerase such as T7 RNA Polymerase. While the single-stranded nucleic acid molecule may be synthesized in vivo or in vitro, the single-stranded nucleic acid molecule is preferably synthesized in vitro because of the easy purification of in vitro products and the availability of modified nucleotides as nucleotides employed.

The nucleotides used when the single-stranded nucleic acid molecule is synthesized in vitro may be commercially available nucleotides or nucleotides synthesized by known methods from commercially available nucleotides. For example, variously modified nucleotides can be produced according to the methods described in the following publications: Blommers et al. (Blommers et al., Biochemistry (1998), 37, 17714-17725.), Lesnik et al. (Lesnik, E.A. et al., Biochemistry (1993), 32,7832-7838.), patent document (US6261840), Martin (Martin, P. Helv. Chim. Acta. (1995) 78,486-504.), patent document (WO1999 / 14226), patent document (WO2000 / 47599), Huynh et al. (Huynh Vu et al., Tetrahedron Letters, 32,3005-3008 (1991)), Radhakrishnan et al. (Radhakrishnan P. lyer et al., J. Am. Chem. Soc. 112, 1253 (1990)), patent document (PCT / WO1998 / 54198), reference document (Oligonucleotide Synthesis, Edited by M. J. Gait, Oxford University Press, 1984), and patent document (Japanese Laid-Open Patent Publication. No. 1995-87982). Each entire disclosure of the publications is incorporated herein by reference.

The synthesized single-stranded nucleic acid molecule may be purified using known methods and devices such as extraction using solvents or resins, precipitation, electrophoresis, and chromatography. The single-stranded nucleic acid molecule may be obtained with the help of manufacturing services by manufacturing companies such as Gene Design, Dharmacon, QIAGEN, and Sigma-Aldrich.

The single-stranded nucleic acid molecule is preferably RNAgiven binding to mRNA which is a transcript of a gene of interest.

When the single-stranded nucleic acid molecule is RNA, it may be designed to be synthesized in a subject applied. In this case, examples of the single-stranded nucleic acid molecule include a vector in which a gene (DNA fragment) encoding the single-stranded nucleic acid molecule is inserted and which is suitable for the subject applied. When such a vector is administrated into the subject, the gene encoding the single-stranded nucleic acid molecule is transcribed in the subject, and then the single-stranded nucleic acid molecule can be synthesized in the subject. Examples of a vector type that can be used for the purpose include, but are not limited to, viral vectors such as retrovirus, lentivirus, adenovirus, and adeno-associated virus (AAV).

The single-stranded nucleic acid molecule can be used in vivo or in vitro. A subject with the single-stranded nucleic acid molecule applied in vivo is not particularly limited and may be any living individuals including animals, plants, and microorganisms. Examples of animals include mammals, and examples of mammals include humans, dogs, cats, cows, horses, pigs, and sheep. Among them, the subject is preferably humans. While the subject applied may be a healthy living individual, it is preferably a living individual which is desired to prevent or treat a genetic disease, for example, a living individual developing or being at the risk of developing the genetic disease selected from those described below. Furthermore, while the subject applied may be a living individual itself, it may be part of a living individual, for example, a cell, a tissue, or an organ.

If the single-stranded nucleic acid molecule is used in vitro, it may be used as a reagent for but is not limited to, experiments, research, and medical treatment. For example, for medical purposes, stem cells can be collected from a living individual suffering from a genetic disease and then modified such that the single-stranded nucleic acid molecule is constantly synthesized. The modified stem cells can be cultured and then transplanted to a living individual suffering from a genetic disease. As such, the single-stranded nucleic acid molecule according to one embodiment of the present invention can be used for medical purposes including regenerative medicine and cell therapy.

The single-stranded nucleic acid molecule is preferably used together with adjuvants for nucleic acid transferring so that it can be easily transferred into the cells of the subject applied. Examples of adjuvants for nucleic acid transferring include, but are not limited to, lipofectamine, oligofectamine, liposome, polyamine, DEAE dextran, calcium phosphate, dendrimer, and lipid nanoparticles.

The amount of the single-stranded nucleic acid molecule used is not particularly limited, so long as the -1 frameshift is induced by using the single-stranded nucleic acid molecule. Examples of the amount used for adults include the range between 0.001 nmol/kg/day and 100 mmol/kg/day, and in terms of having an excellent -1 frameshift-inducing effect as well as the synthesis cost of the single-stranded nucleic acid molecule, preferably the range between 500 nmol/kg/day and 500 mmol/kg/day, and more preferably the range between 40 nmol/kg/day and 500 µmol/kg/day.

While a method for evaluating a -1 frameshift-inducing effect of the single-stranded nucleic acid molecule is not particularly limited, examples of the method include methods including confirming increase or decrease in the amount of mRNA of a gene of interest, in the amount of protein encoded by a gene of interest, or in both amounts, under the presence or absence of application of the single-stranded nucleic acid molecule. The -1 frameshift-inducing effect of the single-stranded nucleic acid molecule may be also evaluated by diagnosing and/or examining to prevent or treat diseases or to alleviate symptoms of the subject with the single-stranded nucleic acid molecule applied.

While the degree of the -1 frameshift-inducing effect of the single-stranded nucleic acid molecule is not particularly limited, the degree is, for example, preferably to the extent that the change in mRNA amount and/or protein amount (increase or decrease in amount) associated with a gene of interest is observed when compared with that in the case that the single-stranded nucleic acid molecule is not used (control).

### [Composition]

The single-stranded nucleic acid molecule is used either alone or in the form of a composition mixed with other components. Another aspect of the present invention is a composition to be used for inducing -1 frameshift, containing the single-stranded nucleic acid molecule of one embodiment of the invention as an active ingredient. Specific embodiments of the composition include pharmaceutical compositions, food and drink compositions, quasi-drug compositions, cosmetic compositions, and animal feed compositions. The composition may take any formulation, for example, oral formulations such as a tablet, capsule, granule, powder, and syrup, as well as parenteral formulations such as injection, suppository, patch, and external preparation.

The other components are not particularly limited, so long as the -1 frameshift is induced when the single-stranded nucleic acid molecule is applied. Examples of the other components include components used in producing pharmaceuticals, and specifically include excipient agents (e.g., sugar derivatives such as lactose, sucrose, glucose, mannitol, and sorbitol; starch derivatives such as corn starch, potato starch, pregelatinized starch, and dextrin; cellulose derivatives such as crystalline cellulose; gum arabic; dextran; organic excipient agents such as pullulan; silicate derivatives such as light anhydrous silicic acid, synthetic aluminum silicate, calcium silicate, and magnesium aluminometasilicate; phosphates such as calcium hydrogen phosphate; carbonates such as calcium carbonate; inorganic excipient agents such as sulfates including calcium sulfate), lubricants (e.g., metal stearates such as stearic acid, calcium stearate, and magnesium stearate; talc; colloidal silica; beads wax; waxes such as sperm whale wax; boric acid; adipic acid; sulfates such as sodium sulphate; glycol; fumaric acid; sodium benzoate; DL leucine; lauryl sulfates such as sodium lauryl sulfate and magnesium lauryl sulfate; silicic acids such as anhydrous silicic acid and silicic acid hydrate; the starch derivatives above, etc.), binding agents (e.g., hydroxypropyl cellulose, hydroxypropylmethyl cellulose, polyvinylpyrrolidone, macrogol, compounds same as the excipient agents above, etc.), disintegrating agents (e.g., cellulose derivatives such as low-substituted hydroxypropyl cellulose, carboxymethyl cellulose, carboxymethyl cellulose calcium, and internally crosslinked sodium carboxymethyl cellulose; chemically modified starch/cellulose such as carboxymethyl starch, sodium carboxymethyl starch, and crosslinked polyvinylpyrrolidone, etc.), emulsifying agents (e.g., colloidal clays such as bentonite and Veegum; metal hydroxides such as magnesium hydroxide and aluminum hydroxide; anionic surfactants such as sodium lauryl sulfate and calcium stearate; cationic surfactants such as benzalkonium chloride; nonionic surfactants such as polyoxyethylene alkyl ether, polyoxyethylene sorbitan fatty acid ester, and sucrose fatty acid ester, etc.), stabilizing agents (paraoxybenzoic acid esters such as methylparaben and propylparaben; alcohols such as chlorobutanol, benzyl alcohol and phenylethyl alcohol; benzalkonium chloride; phenols such as phenol and cresol; timerosal; dehydroacetic acid; sorbic acid, etc.), corrigents (e.g., sweetener, acidifier and flavoring agent commonly used, etc.) , and dilution agent.

The composition may be supplied in the form of a solution. In this case, the composition is preferably stored under refrigeration, frozen conditions, or lyophilized conditions giving the storage stability of the single-stranded nucleic acid molecule. The composition lyophilized may be used by dissolving in a solvent (such as distilled water for injection) to return in the form of a solution when used.

The amount of the composition applied is not particularly limited, so long as the -1 frameshift-inducing effect is achieved. Examples of the amount include about 0.1 mg/day to 10,000 mg/day, preferably 1 mg/day to 1,000 mg/day when applied for adults (60 kg). The composition according to one embodiment of the present invention can be applied once or several times a day. If it is intended that the -1 frameshift inducing effect of the composition according to one embodiment of the present invention is continuously achieved, the composition may be preferably applied for several days, several weeks, several months, or several years continuously or intermittently. Specific usages and dosages of the composition according to one embodiment of the present invention may be appropriately varied depending on the type of a living individual applied; the type and degree of a disease or symptom that a living individual develops or is at risk of developing; the age of a living individual; the administration method to a living individual.

The composition according to one embodiment of the present invention can prevent and/or treat various genetic diseases by inducing -1 frameshift to increase or reduce the expression of a gene of interest. The composition according to one embodiment of the present invention may be preferably a pharmaceutical composition for preventing and/or treating a genetic disease caused by impaired expression of a gene of interest by inducing -1 frameshift to recover the expression of the gene of interest having the target sequence. While the genetic disease is not particularly limited, examples of the genetic disease include muscular dystrophy, cancer, multiple sclerosis, spinal muscle atrophy, Rett syndrome, amyotrophic lateral sclerosis, fragile X syndrome, Prader-Willi syndrome, xeroderma pigmentosum, porphyria, Werner syndrome, fibrodysplasia ossificans progressiva, infantile neuronal ceroid lipofuscinosis, Alzheimer's disease, Tay-Sachs disease, degenerative neurological disorder, Parkinson's disease, rheumatoid arthritis, graft-versus-host disease, arthritis, hematopoietic disease, von Willebrand disease, ataxia-telangiectasia, thalassemia, kidney stone, osteogenesis imperfecta, liver cirrhosis, neurofibromatosis, bullous disease, lysosomal storage disease, Hurler syndrome, cerebellar ataxia, tuberous sclerosis complex, familiar polycythemia, immunodeficiency, kidney disease, lung disease, cystic fibrosis, familial hypercholesterolemia, pigmentary retinopathy, amyloidosis, atherosclerosis, giantism, dwarfism, hypothyroidism, hyperthyroidism, aging, obesity, diabetes, Niemann-Pick disease, and Marfan syndrome. As described herein, it can be achieved to treat and/or prevent genetic disease by administrating an effective amount of the single-stranded nucleic acid molecule or the composition according to one embodiment of the present invention to a living individual developing or being at risk of developing a genetic disease caused by the impaired expression of a gene of interest. Furthermore, it can be also achieved to improve the impaired expression of a gene of interest on a cell, tissue, organ, or living individual itself by administrating an effective amount of the single-stranded nucleic acid molecule or the composition according to one embodiment of the present invention.

Among the genetic diseases, in genetic diseases caused by out-of-frame mutation of exons in a gene, the exons with the out-of-frame mutation are not partially or completely expressed due to the shifting of the reading frame of the exons. As a result, NMD is activated, and the expression level of the gene often decreases. In this case, the use of the composition according to one embodiment of the present invention allows the exons with out-of-frame mutation to be partially or completely expressed by inducing -1 frameshift and correcting the shift of the reading frame of the exons of the gene to cause in-frame mutation. As a result, it can be achieved that the influence of NMD decreases, and the expression level of the gene increases. Examples of such genetic diseases caused by the decrease in the expression level of a gene include muscular dystrophy, cystic fibrosis, xeroderma pigmentosum, β-thalassemia, and colorectal cancer.

On the other hand, the possibility is that a mutant gene that for some reason had not been originally expressed or the expression level of which had been low becomes expressed, resulting in genetic diseases. In this case, the composition according to one embodiment of the present invention can allow the exons of the mutant gene to be partially or completely not expressed by inducing -1 frameshift and shifting the reading frame of the exons of the mutant gene to cause out-of-frame mutation. As a result, it can be achieved that NMD is introduced and the expression level of the harmful mutant gene decreases. Examples of such genetic diseases caused by the synthesis of abnormal protein brought about by the expression of a mutant gene include dominant genetic diseases with high frequency such as cancer, fibrodysplasia ossificans progressiva, familial Alzheimer's disease, familial amyloid polyneuropathy, and Huntington's disease.

For example, muscular dystrophy is caused by the absence or low level of expression of the dystrophin gene. The dystrophin gene consists of 79 exons. Duchenne muscular dystrophy (DMD) patients often have an out-of-frame mutation type dystrophin gene lacking any one or several exons from the 45th exon to the 55th exon (exon 45 to exon 55). GM03429 cells, fibroblasts derived from DMD patients used in the Examples described below, have a mutated dystrophin gene lacking six exons composed of exon 45 to exon 50 (871 nucleotides = 290 x 3 + 1 nucleotides). The lost six exons are composed of 871 nucleotides, which are composed of nucleotides with a multiple of three as well as one nucleotide and cause the shortage of two nucleotides required for in-frame on a sense strand. In other words, the deletion of one nucleotide causes the shortage of two nucleotides insufficient for three nucleotides thereby shifting the reading frame by +1 (backward to the 3' direction). To solve this problem, it is required to shift the reading frame by -1 (forward to the 5' direction), or by +2 (backward to the 3' direction) to form nucleotides with a multiple of three. This is illustrated in Scheme (IV).

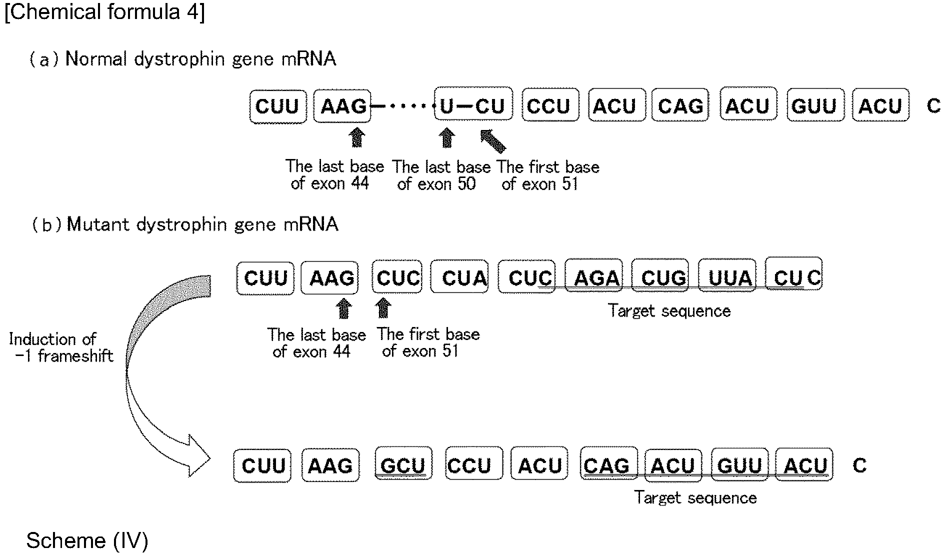

In mRNA of the original dystrophin gene, the last nucleotide of exon 50, U, forms one sense codon UCU (encoding serine) with the beginning two nucleotides of exon 51, CU, and the subsequent CCU of exon 51 forms one sense codon (encoding proline). However, since mRNAof the mutated dystrophin gene lacking exon 45 to exon 50 lacks the last nucleotide of exon 50, U, and the shift of reading frame occurs, the beginning three nucleotides of exon 51, CUC, forms one sense codon (encoding leucine), and the subsequent CUA of exon 51 forms one sense codon (encoding leucine). As a result, the subsequent nucleotide sequence encodes a different amino acid sequence from that of the original dystrophin protein.

On the other hand, it is considered that the composition according to one embodiment of the present invention can be used to induce -1 frameshift to cause ribosome slipping by designating the underlined position indicated in Scheme (IV) as the target sequence. In other words, the sense codon GCU is formed after the last sense codon (AAG) of exon 44 because of -1 frameshift, and subsequently, the triplet codons are formed. As a result, the same amino acids as those of mRNA of the original dystrophin gene would be encoded from the second sense codon of exon 51 (CCU).

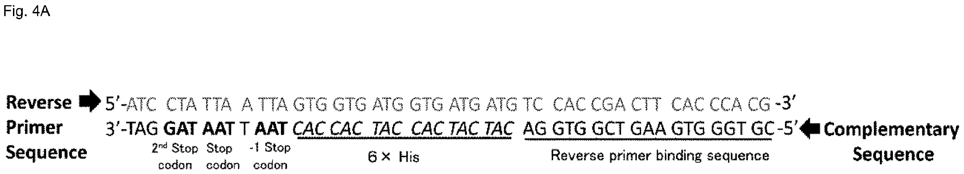

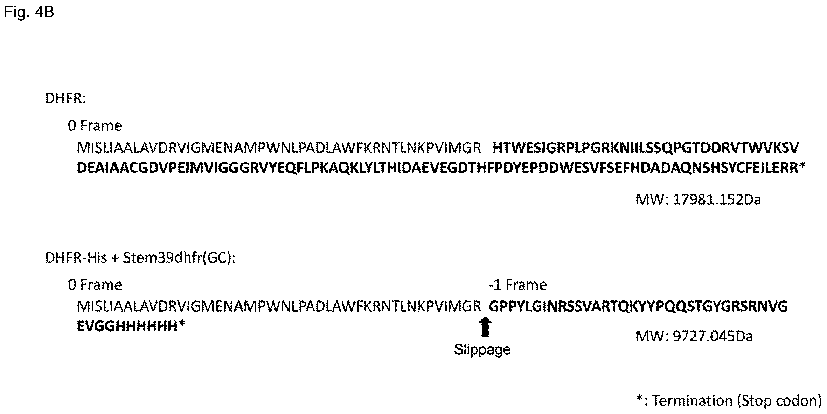

In this case, as a result, -1 frameshift allows mRNA of the mutated dystrophin gene to produce a protein having alanine encoded by the sense codon (GCU) instead of serine encoded by the sense codon (UCU) formed from the last nucleotide of exon 50 and the two nucleotides at the beginning of exon 51. It is noted that these presumptions are based on the data obtained from experiments, and the number of nucleotides between the target sequence and the induction position of -1 frameshift may be appropriately changed. In addition, this theory on -1 frameshift almost coincides with prior models relating to -1 frameshift advocated by Jacks et al. and Weiss et al. as described in Fig.4 of FARABAUGH (PHILIP J. FARABAUGH, MICROBIOLOGICAL REVIEWS, Mar. 1996, p. 103-134; the entire disclosure of which is incorporated herein by reference).

Based on the presumptions, by using the composition according to one embodiment of the present invention, the amino acid sequence of the mutated dystrophin protein expressed by the mutated dystrophin gene is different from that of the normal dystrophin protein only in terms of amino acids encoded by from exon 45 to exon 50 and an amino acid encoded by the sense codon formed by the last nucleotide of exon 50 and the two nucleotides at the beginning of exon 51 (alanine instead of serine) while the remaining amino acid sequence is the same amino acid sequence as that of the normal dystrophin protein. Therefore, the composition according to one embodiment of the present invention can be used to express the mutated dystrophin protein having the amino acid sequence different from that of the normal dystrophin protein only by a single amino acid except for the portions encoded by the exons deleted.

As another embodiment, it is expected to stagnate or alleviate the progression of cancers by changing amino acid sequences of expression products of oncogenes such as c-myc gene, or by reducing the oncogene expression level. Thus, the composition according to one embodiment of the present invention can be used to prevent and/or treat cancers by inducing -1 frameshift targeting mRNA which is a transcript of an oncogene to change the structure of expression product of the oncogene to reduce the activity or expression level of a product expressed by the oncogene. In addition, c-myc gene is used in producing so-called "induced pluripotent stem cells" (iPS cells). Thus, it is expected to produce iPS cells and prevent the resulting iPS cells from becoming cancerous by using the composition according to one embodiment of the present invention targeting c-myc gene used in producing iPS cells at the same time as or at different times from the introduction or expression of the c-myc gene.

While a method of producing the composition according to one embodiment of the present invention is not particularly limited, examples of the method include the method of mixing the single-stranded nucleic acid molecule as an active ingredient with any other components and forming a desired dosage form. The packaging form of the composition according to one embodiment of the present invention is not particularly limited and may be appropriately selected depending on the dosage form applied. Examples of the packaging form include glass containers such as vials and ampules; vials, ampules, bottles, cans, pouches, blister packs, strips, one-layer or laminated film bags, made of materials such as glass, metal such as aluminum, coating paper, and plastic such as PET.

As a non-limiting specific embodiment, the composition according to one embodiment of the present invention can be applied to a DMD patient in the following manner. The single-stranded nucleic acid molecule as an active ingredient is produced by known methods, sterilized by conventional methods, and, for example, 1 ml of an injectable solution containing the single-stranded nucleic acid molecule is prepared. The injectable solution is intravenously administered by infusion such that the dose of the single-stranded nucleic acid molecule is in the range between 0.1 mg and 100 mg per 1 kg of body weight of the patient. Examples of the administration include administrations repeated several times at intervals of 1 week to 2 weeks, and subsequent administrations repeated appropriately while evaluating a muscle strengthening effect by diagnosis; imaging of X-ray, CT, and MRI; observation with endoscope and laparoscope; cytology and tissue diagnosis.

The present invention will now be described in further detail with reference to Examples, which are not intended to limit the present invention. The present invention may take various embodiments to the extent that the objectives of the present invention are achieved.

### Examples

### [Example 1. Evaluation of -1 frameshift of ORF of EGFP-Human α-Tubulin gene]

### 1. Summary of evaluation

ORF (Open Reading Frame) of EGFP-Human α-Tubulin in pEGFP-Tub vector introduced into 143Btk- osteosarcoma cells was intentionally subjected to -1 frameshift using a nucleic acid molecule created artificially. The nucleic acid molecule induced -1 frameshift of ORF at a position around a target sequence to make the amino acid sequence of EGFP fluorescent protein change in the middle, thereby causing Premature Termination Codon (PTC) as a first stop codon (TGA) at the boundary between EGFP gene sequence and α-Tubulin gene sequence.

### 2. Preparation of test cell line

143Btk- osteosarcoma cells were cultured in DMEM supplemented with 10% FCS and then were transfected with pEGFP-Tub vector (CLONTECH Laboratories; 6.0 kb) using the transfection reagent "Lipofectamine 2000" (Thermo Fisher Scientific). Clones of 143Btk-osteosarcoma cells in which EGFP fluorescent protein fused with human α-Tubulin protein at the C-terminal was highly expressed stably were selected as a test cell line by drug selection using G418 sulfate (Cosmo Bio).

### 3. Preparation of nucleic acid molecule for frameshifting

For the nucleic acid molecule inducing -1 frameshift, several types of nucleic acid molecules were molecularly designed based on the conditions described herein. Each conformation of them in a reaction solution was simulated by the RNA secondary structure prediction program "CentroidFold" (obtained from: https://ghub.com/satoken/centroid-rna-package), and the nucleic acid molecules with a highly stable conformation were obtained by custom synthesis.

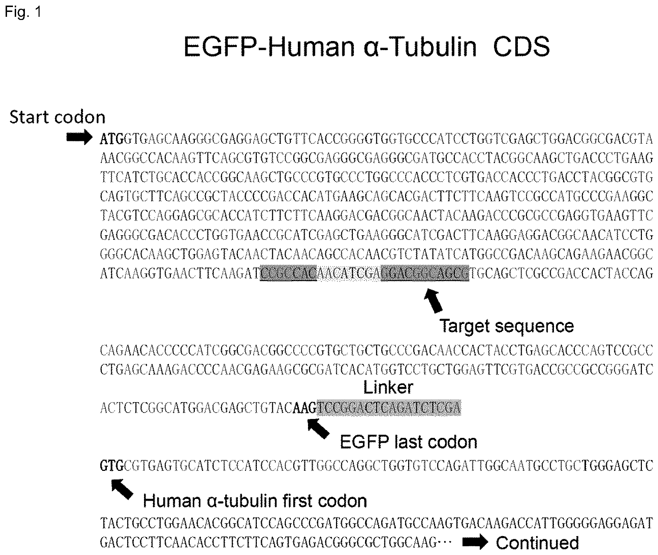

The coding sequence (CDS) of the EGFP-Human α-Tubulin is shown in Fig. 1. As shown in Fig. 1, the EGFP gene (SEQ ID NO: 7) has been linked to the Human α-Tubulin gene (SEQ ID NO: 9) via the linker (5'-TCCGACTCAGATCTCGA-3'; SEQ ID NO: 8).

The molecular design was carried out based on a position of a target sequence. Specifically, the target sequence was designed to have ten and several nucleotides downstream of two nucleotides corresponding to the bottom of the axis of a stem-loop via a spacer sequence positioned six nucleotides away from the position of the last original codon in which slipping occurs. The target sequence was also designed not only to bind enough to the nucleic acid molecule to the extent that the nucleic acid molecule for frameshifting could become an obstacle to the movement of the ribosome to cause mechanistic slipping by -1 nucleotide at the upstream sequence but to bind loosely to the nucleic acid molecule for frameshifting to the extent that the nucleic acid molecule could be detached from mRNA after -1 nucleotide slipping to resume translation. For this reason, the target sequence was preferably composed of ten and several nucleotides, and the length of the target sequence was determined such that the sequence of the molecule to bind to the target sequence could not be bound to or interacted with the other sequence of the molecule and the stem-loop structure of the nucleic acid molecule for frameshifting could not collapse. Experimentally, the target sequence was designed to have a length in the range between 10 nucleotides and about 13 nucleotides. The nucleic acid molecule for frameshifting was designed to have the most stable structure by adjusting the chain length while confirming that the basic stem-loop structure could not collapse by using CentroidFold which is a secondary structure prediction program for RNA. Consequently, it was inferred that the nucleic acid molecule for frameshifting had a more stable molecular structure for the target sequence.

Theoretically, the designed nucleic acid molecule for frameshifting causes -1 frameshift at a position around the target sequence thereby forming PTC around the boundary between the EGFP gene sequence and the human α-tubulin gene sequence. As a result, only EGFP fluorescent protein can be expressed with α-tubulin protein deleted in 143Btk- osteosarcoma cells. In addition, since the structure of pre-mRNA for a fluorescent protein expressed by the pEGFP-Tub vector is made of a single exon, NMD (Nonsense-mediated mRNA decay), which is one of the intracellular mRNA surveillance mechanisms, cannot be activated as shown in Example 3 described below. In other words, the mRNA expression of the EGFP gene still exists.

The nucleic acid molecule for frameshifting, "Stem38egfp (CG)" (SEQ ID NO: 1), was designed for the target sequence. In addition, the nucleic acid molecule, "acGFP2" (SEQ ID NO: 6), was also designed, which has a similar sequence or structure to Stem38egfp (CG) but does not target the EGFP gene. The structures of Stem38egfp (CG) and acGFP2 are shown in Schemes (V) and (VI), respectively.

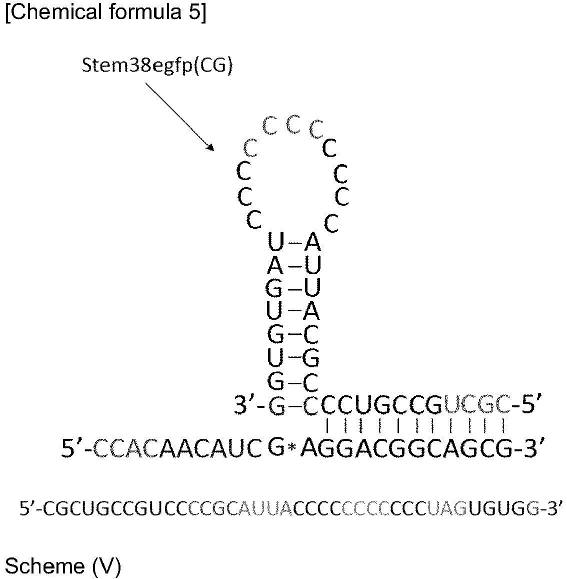

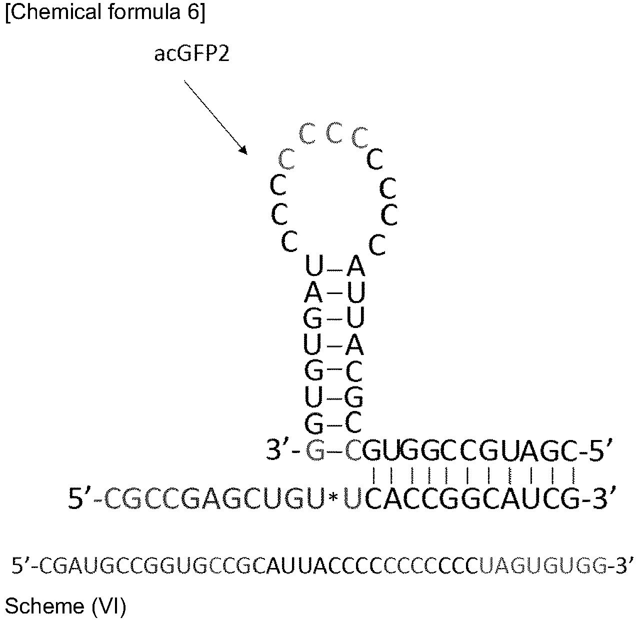

The nucleic acid molecules were dissolved in TE buffer to prepare each 100 µM nucleic acid molecule solution.

### 4. Induction of frameshifting

The test cell line was seeded in each well of 6-well plates and then cultured at 37 °C under 5% CO₂ for 2 days. After reaching 80% confluency, the medium in each well was replaced with Opti-MEM^{®} (Thermo Fisher Scientific) supplemented with a mixture of the nucleic acid molecule and the transfection reagent ("Lipofectamine 2000"). After the cells were subjected to transfection treatment for 6 hours in the above Opti-MEM, the 6-well plates were placed for chemical treatment by incubating at 37 °C under 5% CO₂ for 48 hours and then subjected to assay.

The following five test groups were prepared: untreated (Unt) group with Opti-MEM only without adding any nucleic acid molecule and transfection reagent; Mock group with Opti-MEM supplemented with only the transfection reagent without adding any nucleic acid molecule; P group with the molecule "Stem39egfp (GC)" targeting the target sequence; and NC (Negative Control) group with the molecule "acGFP2" not targeting EGFP gene. In the P group and NC group, the final concentration of each nucleic acid molecule in Opti-MEM was set to become 40 nM.

After the transfection treatment, the medium in each well was replaced with DMEM supplemented with 15% FCS, and the 6-well plate was incubated at 37 °C under 5% CO₂ for 48 hours. After the incubation, the cells in each well were observed using the fluorescence microscope "BZ-X9000" (Keyence) to confirm stably expressed green fluorescent proteins that were transcribed and translated by the pEGFP-Tub vector.

### 5. Evaluation of frameshift induction

Fig. 2 shows the results of observing the cells in each test group with the fluorescence microscope. As shown in Fig. 2, fluorescence was observed in the cytoplasm excluding the nucleus in the cells of the Unt group, Mock group, and NC group since human α-Tubulin protein-fused EGFP proteins were oriented along the cytoskeleton. On the other hand, in the cells of the P group, weak fluorescence, which appeared to be diffused and smeared, was observed.

From the observation results, it was found that the nucleic acid molecule Stem38egfp (CG) used in the P group induced -1 frameshift to shift the reading frame; the shift of the reading frame allowed human α-Tubulin, which was one of the scaffold proteins, to be deleted from EGFP-human α-Tubulin fusion protein, and thus EGFP protein failing with its orientation was randomly diffused into the cells; and from the observation of weak fluorescence, EGFP protein expressed had the amino acid sequence that was changed from the portion in which the shift of ORF occurred to make fluorescence weakened as a whole when compared with that of the original amino acid sequence.

### [Example 2. Evaluation of -1 frameshift of ORF of DHFR gene]

### 1. Summary of evaluation

DNA fragments containing two types of dihydrofolate reductase (DHFR) genes were used to verify the -1 frameshift of ORF through in vitro cell-free translation system.

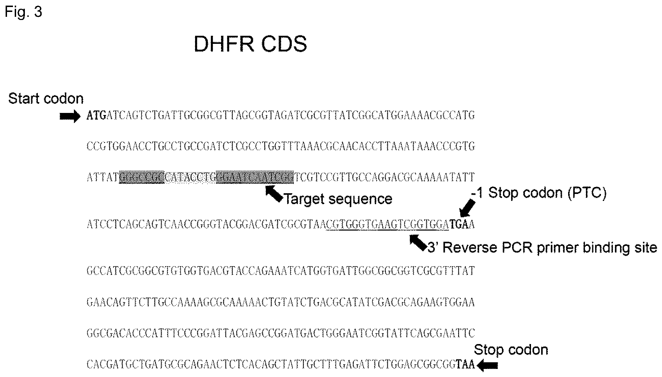

Fig. 3 shows the CDS of the DHFR gene (SEQ ID NO: 10) derived from *Escherichia coli.* DHFR, which is the first DNA fragment, contains a T7 promoter and ribosome binding site (SD sequence) located upstream of the start codon (ATG) of the DHFR gene.

DHFR is transcribed and translated to obtain an original DHFR protein. On the other hand, when -1 frameshift occurs, the reading frame is shifted to form -1 stop codon (PTC) as shown in Fig. 3, and the translation is terminated. As a result, a DHFR protein in the incomplete length encoded by the sequence before PTC is obtained.

DHFR-His, which is a second DNA fragment, is a DNA fragment obtained by PCR using the first DNA fragment as a template and a pair of the forward primer "Primer DHFR-His_Fw" (SEQ ID NO: 11) complementary to a part of the sequence located upstream of T7 promoter and a part of T7 promoter sequence and the reverse primer "Primer DHFR-His_Rv" (SEQ ID NO: 12) complementary to "3' Reverse PCR primer-binding site" (Fig. 3) in the middle of DHFR gene. The nucleotide sequence of the reverse primer is shown in Fig. 4A.

As shown in Fig. 4A, "Primer DHFR-His_Rv" has a sequence configuration to form a polyhistidine-tag (His-tag) by -1 frameshifting. Normally, when DHFR-His is transcribed and translated, the translation proceeds until stop codons (TAA, TAG) are located on the 3' side of the reverse primer, and a protein with no His-tag is obtained. On the other hand, when a -1 frameshift occurs to shift the reading frame, the translation terminates at the -1 stop codon (TAA), and a protein with His-tag at the C-terminal is obtained.

As described above, when DHFR and DHFR-His are employed, different proteins can be obtained depending on whether -1 frameshift occurs or not. Fig. 4B shows the amino acid sequence of DHFR protein (SEQ ID NO: 13) and the amino acid sequence of DHFR-His protein (SEQ ID NO: 14). When such DNA fragments are used through a reconstituted cell-free translation system, -1 frameshift can be detected by binding the artificially designed nucleic acid molecule to the target sequence in DHFR gene and by shifting the original ORF by -1 nucleotide to change the amino acid sequence of protein synthesized.

### 2. Preparation of nucleic acid molecule for frameshifting

The target sequence was determined by selecting a position around a predicted site where slippage of -1 nucleotide easily would occur. Fig. 3 shows the location of the target sequence on the DHFR gene.

As mentioned above, it is predicted that if DHFR-His is transcribed and translated in a state of causing -1 frameshift, a protein of about 9.6 kDa fused with His-tag, which is encoded by a sequence immediately before a firstly appeared stop codon (-1 stop codon (PTC)), at the C-terminal is synthesized. The nucleic acid molecule for frameshifting with a stable molecular structure was prepared as "Stem39dhfr (GC)" (SEQ ID NO: 2). The structure of Stem39dhfr (GC) is shown in Scheme (VII).

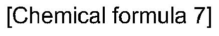

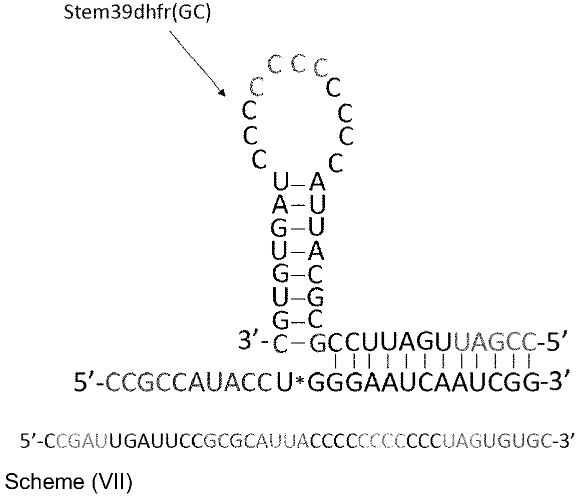

### 3. Induction of frameshifting

The in vitro cell-free translation kit "PUREflex ^{®} 2.0" (Cosmo Bio) was used. As the DHFR gene, "DHFR DNA" attached to the kit was used. DHFR-His was obtained by carrying out PCR using DHFR-His_Fw and DHFR-His_Rv and using DHFR as a template according to conventional methods, and the obtained PCR product was purified.

The reaction solutions attached to the kit used are listed in Table 1. In Table 1, the sample solutions were used as shown in Table 2.

**[Table 1]**

| | ×1 |
|---|---|
| Solution I | 10.0µL |
| Solution II | 1.0µL |
| Solution III | 2.0µL |
| Sample solution | 7.0µL |
| Total | 20.0µL |

**[Table 2]**

| Test group | 1 | 2 | 3 | 4 | 5 |
|---|---|---|---|---|---|
| Name | NC | PC | TEST1 | TEST2 | PN |
| Stem39dhfr(GC) | (-) | (-) | (+) | (+) | (-) |
| DHFR | (-) | (+) | (+) | (-) | (-) |
| DHFR-His | (-) | (-) | (-) | (+) | (+) |

For Negative Control (NC), distilled water (ddH₂O) was used as the sample solution (Test group 1). For Positive Control (PC), the solution containing the DHFR gene having the full length of the DHFR gene was used as the sample solution (Test group 2). For frameshift reaction group 1, the solution containing the nucleic acid molecule for frameshift Stem39dhfr (GC) and DHFR was used as the sample solution (Test group 3). For frameshift reaction group 2, the solution containing Stem39dhfr (GC) as well as both parts of the DHFR gene and DHFR-His designed to encode His-tag by -1 frameshift was used as the sample solution (Test group 4). For Positive Negative Control (PN), the solution containing only DHFR-His was used as the sample solution (Test group 5). Each sample solution containing Stem39dhfr (GC) was prepared such that the concentration of Stem39dhfr (GC) in the reaction solution (20 µl) became 500 nM. In the same manner, each sample solution containing DHFR and/or DHFR-His was prepared such that the amounts of DHFR and DHFR-His in the reaction solution (20 µl) became 20 µg and 60 µg, respectively.

Each reaction solution was placed in a tube for thermal cycler and then subjected to protein synthesis reaction using a thermal cycler at 37 °C for 4 hours.

To 20 µL of each reaction solution according to Test groups 1 to 3, 20 µL of ddH₂O was added and diluted two times. To the obtained diluted solution, 13.3 µL of 4 × SDS sample buffer containing 10 vol% β-mercaptoethanol was added, and the mixture was heated at 95 °C for 5 minutes to denature proteins.

To 20 µL of each reaction solution according to Test groups 4 and 5, 275 µL of xTractor buffer attached to the His-tag fusion protein purification kit "Capturem^{™} His-tagged Preparation Miniprep Kit" (Clontech) was added to prepare about 300 µL of solution A. The prepared solution A was loaded to an equilibrated His-tag fused protein adsorption column. Equilibration of the column was carried out by loading xTractor buffer and centrifuging at 9,800 g for 1 minute and 45 seconds.

The column with solution A loaded was centrifuged at 9,800 g for 1 minute and 45 seconds, and the obtained supernatant was collected as "Lysate". Then, 300 µL of wash buffer was loaded into the column. The column was then centrifuged at 9,800 g for 1 minute and 45 seconds, and the obtained supernatant was collected as "Wash". To elute His-tag fused proteins from the column, 300 µL of elution buffer was then loaded into the column. The column was then centrifuged at 9,800 g for 1 minute and 45 seconds, and the obtained supernatant was collected as "Elution 1". The same operation was carried out to collect the supernatant as "Elution 2".

To 150 µL of each collected solution, 45 µL of 4 × SDS sample buffer was added and mixed and then dispensed every 50 µL. The dispensed solution was placed in a tube and heated using a thermal cycler at 95 °C for 5 minutes to denature proteins. The heat-denatured protein samples were stored at -20 °C.

On the day of testing, the heat-denatured protein samples in a frozen state were collected in a 1.5 mL tube every "Lysate", "Wash", "Elution 1", and "Elution 2", respectively. Then, the whole amount of each sample solution was loaded in the filter column "Amicon^{®} Ultra-0.5 (3k)" (Max Volume 500 µL; Merck Millipore), and then the filter column was centrifuged at 12,000 g for about 45 minutes. The filter column was then turned upside down and centrifuged at 12,000 g for 2 minutes to collect a concentrated protein sample from the filter column. The whole amount (45 µL) of the collected concentrated protein sample was applied to each well of the polyacrylamide gel for protein electrophoresis "10-20% Criterion^{™} TGX^{™} precast gel" (Bio-Rad). In a similar manner, each whole amount of the heat-denatured protein samples according to Test groups 1 to 3 was applied to each well thereof. The gel with the protein samples applied was subjected to electrophoresis at 200 V for 50 minutes. Table 3 shows the arrangement of the protein samples applied to each well. The molecular weight marker (M) "Precision Plus Protein Dual Extra Standard" (Bio-Rad Laboratories; product number 161377) was used.

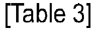

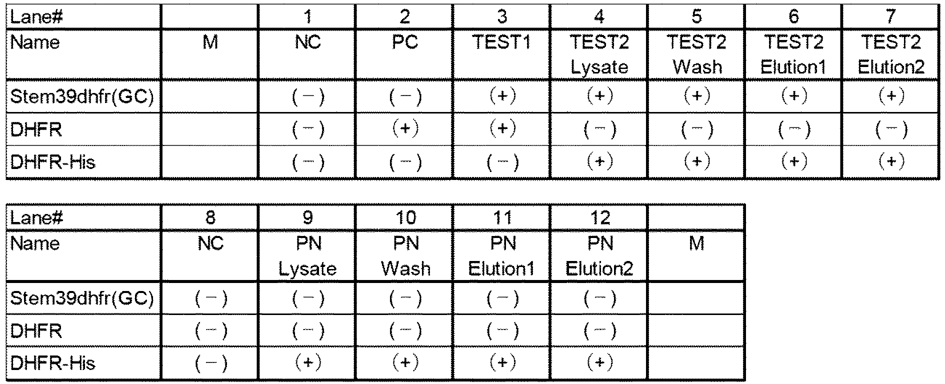

After electrophoresis, the gel was stained with the Coomassie-dye-protein-gel-stain reagent "Simply Blue Safe Stein" (Thermo Fisher Scientific) to visualize each band.

### 4. Evaluation of frameshift induction

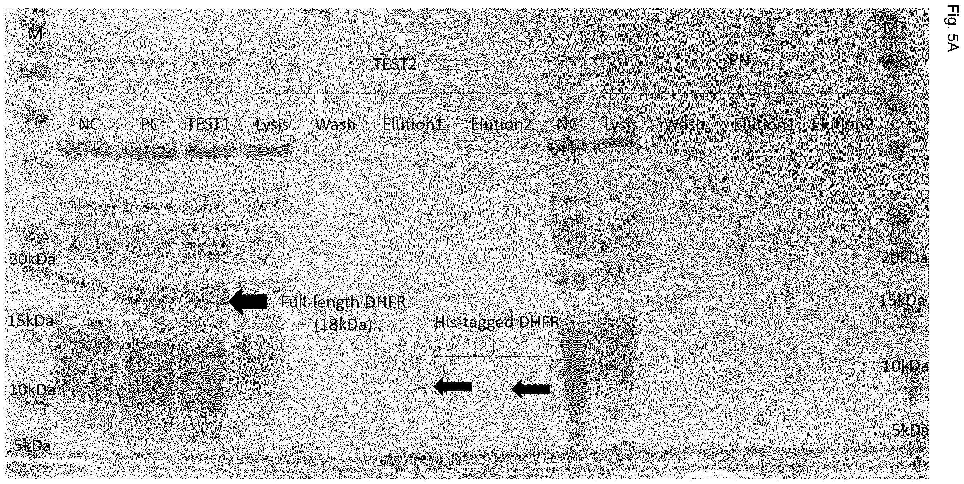

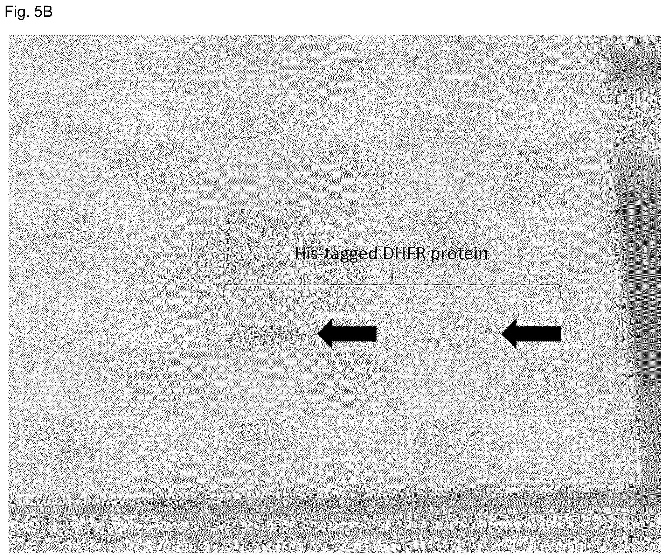

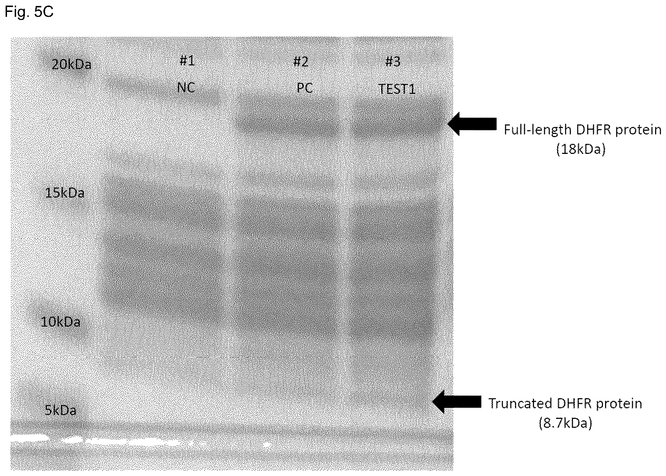

The results of gel staining are shown in Figs. 5A to 5C. Fig. 5A is the picture image of the whole gel after staining. Fig. 5B is part of Fig. 5A expanding the portion at which His-tagged DHFR protein according to TEST 2 Elution 1 and Elution 2 appeared. Fig. 5C is part of Fig. 5A expanding the portion at which DHFR protein and DHFR protein fragments according to NC, PC, and TEST 1 appeared.

The bands of His-tag fused protein were observed in Lane 6 (TEST 2 Elution 1) and Lane 7 (TEST 2 Elution 2) applying the reaction solution (TEST 2) to which DHFR-His and the nucleic acid molecule for frameshift Stem39dhfr (GC) were added. In particular, the clear band was observed in Lane 6. On the other hand, in Lane 11 (PN Elution 1) and Lane 12 (PN Elution 2) applying the reaction solution (PN) to which only DHFR-His was added but the nucleic acid molecule for frameshift Stem39dhfr (GC) was not added, there was no band observed at the position where it would have appeared if the His-tag fused protein had been synthesized.

The results demonstrated that the nucleic acid molecule for frameshift Stem39dhfr (GC) could cause a -1 frameshift of ORF during translation of DHFR-His to synthesize the His-tag fused protein.

On the other hand, as with Lane 2 applying the reaction solution (PC) to which only DHFR was added, the band of DHFR protein in full length was observed in Lane 3 (TEST 1) applying the reaction solution (TEST 1) to which both DHFR and the nucleic acid molecule for frameshift Stem39dhfr (GC) were added. In Lane 3, however, the concentrated band of the partial fragment of DHFR protein (truncated DHFR protein) was observed.

The results demonstrated that the nucleic acid molecule for frameshift Stem39dhfr (GC) could cause -1 frameshift of ORF to form a stop codon that was not present in an original DHFR gene sequence, i.e., PTC, thereby interrupting the translation.

The results proved that the artificially designed nucleic acid molecule for frameshifting could artificially cause -1 frameshift.

### [Example 3. Evaluation of -1 frameshift of ORF of dystrophin gene]

### 1. Summary of evaluation

If ORF is shifted due to deletion of exons and the like and PTC (Premature Termination Codon) is formed in the middle of ORF of mRNA with out-of-frame mutation, NMD (Nonsense-mediated mRNA decay), which is a surveillance mechanism for mRNA, can be induced to preferentially degrade mRNA with PTC.

Duchenne muscular dystrophy (DMD) is caused by the absence of dystrophin protein synthesis due to the partial deletion of 79 exons of the dystrophin gene (HGNC ID HGNC: 2928, Chromosomal location: Xp21.2 to p21.1) and degradation of mRNA with PTC formed. If the nucleic acid molecule for frameshifting can be used to artificially induce -1 frameshift of ORF in living cells derived from patients suffering from genetic diseases like DMD, it is possible to avoid NMD and the degradation of mRNA to synthesize a dystrophin protein in a relatively longer length.

In this example, it was attempted to design as a therapeutic molecule a nucleic acid molecule for frameshifting to target dystrophin mRNA, to transfect the nucleic acid molecule in fibroblasts derived from patients of Duchenne muscular dystrophy lacking exon 45 to exon 50 in the dystrophin gene, and to induce -1 frameshift of ORF of the gene. The possibility that -1 frameshift occurred in living cells thereby treating genetic diseases was demonstrated by confirming to increase in the expression level of mRNA of dystrophin gene by causing -1 frameshift to correct out-of-frame into in-frame during ribosome translation on dystrophin mRNA.

### 2. Test cell line

GM03429 cells, fibroblasts derived from DMD patients, have mutated dystrophin genes lacking six exons (871 nucleotides = 290 × 3 + 1 nucleotides) of exon 45 to exon 50 of the dystrophin gene. GM05118 cells, fibroblasts derived from healthy subjects, have normal dystrophin genes. GM03429 cells and GM05118 cells were obtained from Coriell Institute for Medical Research.

### 3. Preparation of nucleic acid molecule for frameshifting

The mutated dystrophin gene lacks exon 45 to exon 50 resulting in the sequence in which the 3' end (AAG) of exon 44 directly binds to the 5' end (CTC) of exon 51. The breakpoint was set at the boundary between AAG and CTC above.

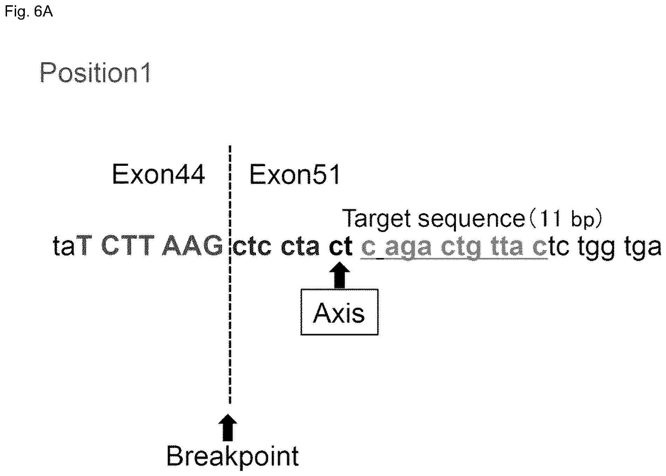

Three types of target sequences, Position1, Position2, and Position3, were set around the breakpoint between exon 44 and exon 51 of the mutated dystrophin gene. For each of them, the nucleic acid molecules for frameshift "P1Stem38dmd45-50 (GC)" (SEQ ID NO: 3), "P2Stem39dmd45-50 (CG)" (SEQ ID NO: 4), and "P3Stem39dmd45-50 (CG)" (SEQ ID NO: 5) were designed and prepared, respectively. The stabilities of the nucleic acid molecules designed for frameshifting were calculated and confirmed using "CentroidFold". The target sequences Position 1, Position 2, and Position 3 are shown in Figs. 6A to 6C. The structures of P1Stem38dmd45-50 (GC), P2Stem39dmd45-50 (CG), and P3Stem39dmd45-50 (CG) are illustrated in Schemes (VIII) to (X), respectively.

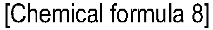

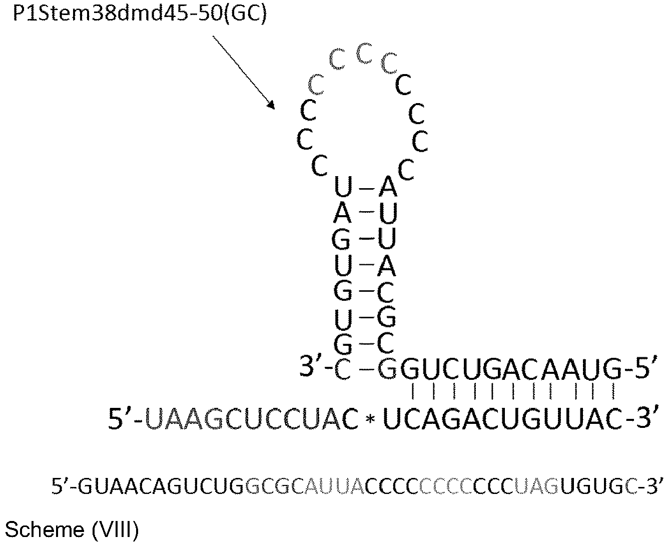

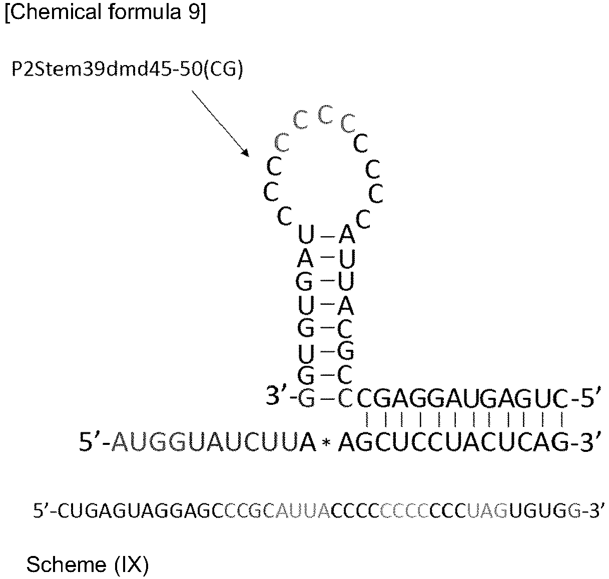

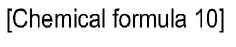

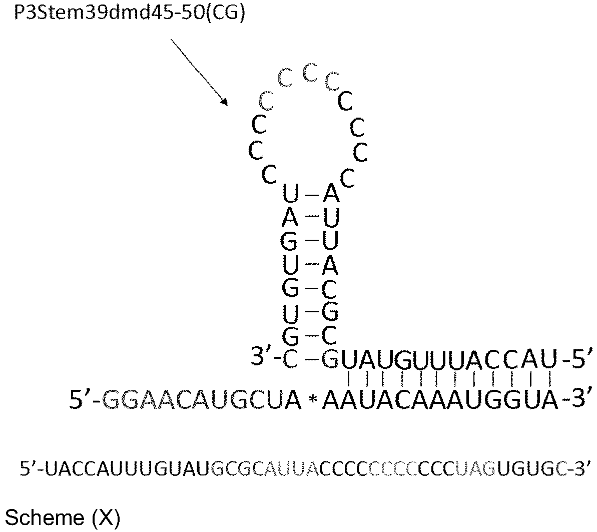

### 4. Cell culture

The test cell lines (GM03429 cells and GM05118 cells) were subjected to passage culture in 6-well plates using DMEM supplemented with 15% FCS and Antibiotic Antimycotic under the conditions of 37 °C and 5% CO₂, respectively. The cells were collected after culturing.

### 5. Induction of frameshift and synthesis of cDNA

The collected test cell lines were seeded in each well of 96-well plates and then cultured at 37 °C under 5% CO₂ for 2 days. After reaching 90% confluency, the medium in each well was replaced with Opti-MEM^{®} (Thermo Fisher Scientific) supplemented with a mixture of the nucleic acid molecule and the transfection reagent ("Lipofectamine 2000"). After 6 hours, the medium was replaced with supplemented DMEM, and after transfection treatment, the 96-well plates were subjected to chemical treatment at 37 °C under 5% CO₂ for 48 hours.

The following five test groups were prepared using GM03429 cells as a test cell line: untreated (Unt) group (n=1) with only Opti-MEM without adding any nucleic acid molecules and transfection reagent; Mock group (n = 6) with Opti-MEM supplemented with only the transfection reagent without adding any nucleic acid molecules; P1 group (n=3) with the molecule "P1Stem38dmd45-50 (GC)" targeting Position 1; P2 group (n=3) with the molecule "P2Stem39dmd45-50 (CG)" targeting Position 2; and P3 group (n=3) with the molecule "P3Stem39dmd45-50 (CG)" targeting Position 3. In addition, using GM05118 cells as a test cell line, a test group using only Opti-MEM without adding any nucleic acid molecules and the transfection reagent was defined as wild type (WT) group (n=2). In the P1, P2, and P3 groups, the final concentration of each nucleic acid molecule in Opti-MEM was set to become 40 nM.

After completion of transfection, the cells were collected from each well and then lysed using the cDNA synthesis kit "SuperScript^{™} III CellsDirect cDNA Synthesis System" (Thermo Fisher Scientific), and a total RNA in the obtained lysate was subjected to reverse-transcription to produce cDNA.

### 6. Quantification of dystrophin mRNA by qPCR

For quantification of dystrophin mRNA, qPCR was carried out by ΔΔCT method using the real-time PCR kits "TaqMan^{®} Gene Expression Master Mix" and "TaqMan^{®} Gene Expression Assay" (Thermo Fisher Scientific) as well as the real-time PCR device "TaKaRa PCR Thermal Cycler Dice Real Time System II". GAPDH gene was used for an endogenous control, and the expression level of dystrophin mRNA was calculated as a relative level concerning the control expression level.

### 4. Evaluation of frameshift induction

Fig.7 shows the dystrophin mRNA expression level relative to the GAPDH mRNA expression level for each test group.

The expression level of dystrophin gene mRNA in the Unt group was lower than that in the WT group using normal fibroblasts. Similarly, the expression level was very low in the Mock group with only the transfection reagent. The results of these test groups suggested that the dystrophin mRNA with PTC formed by the exon deletion was captured by the mRNA surveillance mechanism and degraded by NMD.

On the contrary, it was observed that all P1 group, P2 group, and P3 groups using the nucleic acid molecules for frameshift P1Stem38dmd45-50 (GC), P2Stem39dmd45-50 (CG), and P3Stem39dmd45-50 (CG) directed to Position 1, Position 2, and Position 3 increased the expression level of dystrophin mRNA in DMD patient-derived fibroblasts having the nucleic acid molecules introduced. The results indicated that since the nucleic acid molecule for frameshift bound to and acted on mRNA, out-of-frame of ORF could be changed to in-frame to avoid NMD during translation of ORF by the ribosome. When the nucleic acid molecule for frameshift P2Stem39dmd45-50 (CG) directed to Position 2 was used, the Premature Termination Codon (PTC) of UAA could be formed before the axis (Axis). When the nucleic acid molecule for frameshift P3Stem39dmd45-50 (CG) directed to Position 3 was used, PTC of UAA was formed before the axis (Axis) and after the target sequence. Such a fact might have led to the results that the expression level of mRNA using the P2 group or P3 group was lower than that using the P1 group. Since the expression level of mRNA in the P2 group or P3 group was, however, higher than that of the Mock group, it was found that the nucleic acid molecules for frameshifting used in the above groups could avoid NMD. The finding of NMD is not necessarily induced only due to the occurrence of PTC is a surprising one first found by the present inventor.

From the results, it was found that the nucleic acid molecule for frameshifting can be used to avoid NMD or other problems to prevent dystrophin mRNA from degrading and retain the expression of dystrophin mRNA. As such, it is very surprising that the expression of dystrophin gene mRNA in DMD patient-derived fibroblasts could be confirmed by means of the nucleic acid molecule for frameshifting since the expression level of dystrophin gene mRNA in DMD patient-derived fibroblasts was normally very low. This fact directly leads to the conclusion that the nucleic acid molecule for frameshift can be effectively used for DMD treatment.

As above, the nucleic acid molecule for frameshift artificially designed to induce -1 frameshift can be used for an effective therapeutic method for DMD, namely, Duchenne muscular dystrophy, which is caused by producing very less dystrophin protein due to degradation of dystrophin mRNA.

In a similar manner to Duchenne muscular dystrophy caused by deletions of exon 45 to exon 50 of the dystrophin gene, other genetic disorders caused by a shift of +1 ORF can be also treated with the nucleic acid molecule for frameshifting that induces -1 frameshift.

On the other hand, it is possible for the nucleic acid molecule for frameshifting to induce - 1 frameshift of ORF of mRNA of a target gene functioning in cells to give rise to incorrect translation of mRNA and form an intentional PTC on mRNA, resulting in activating NMD to urge mRNA to degrade thereby reducing expression of mRNA, and changing a functional protein into a nonfunctional truncated protein, and as a result, nullifying the function of the target gene in cells.

Since the nucleic acid molecule for frameshifting can be designed for various genes and nullify functions of various genes, for example, genes specifically expressed in cancer cells, the nucleic acid molecule for frameshifting can be also used for the treatment of diseases such as cancers and dominant genetic diseases caused by expressions of harmful proteins in a living body.

The sequences listed in the sequence listing are as follows:
[SEQ ID NO: 1] Stem38egfp (CG)
   CGCUGCCGUCCCCGCAUUACCCCCCCCCCCUAGUGUGG
[SEQ ID NO: 2] Stem39dhfr (GC)
   CCGAUUGAUUCCGCGCAUUACCCCCCCCCCCUAGUGUGC
[SEQ ID NO: 3] P1Stem38dmd45-50 (GC)
   GUAACAGUCUGGCGCAUUACCCCCCCCCCCUAGUGUGC
[SEQ ID NO: 4] P2Stem39dmd45-50 (CG)
   CUGAGUAGGAGCCCGCAUUACCCCCCCCCCCUAGUGUGG
[SEQ ID NO: 5] P3Stem39dmd45-50 (CG)
   UACCAUUUGUAUGCGCAUUACCCCCCCCCCCUAGUGUGC
[SEQ ID NO: 6] acGFP2
   CGAUGCCGGUGCCGCAUUACCCCCCCCCCCUAGUGUGG
[SEQ ID NO: 7] EGFP gene 
ATGGTGAGCAAGGGCGAGGAGCTGTTCACCGGGGTGGTGCCCATCCTGGTCGAGCTGGAC
GGCGACGTAAACGGCCACAAGTTCAGCGTGTCCGGCGAGGGCGAGGGCGATGCCACCTAC
GGCAAGCTGACCCTGAAGTTCATCTGCACCACCGGCAAGCTGCCCGTGCCCTGGCCCACC
CTCGTGACCACCCTGACCTACGGCGTGCAGTGCTTCAGCCGCTACCCCGACCACATGAAGC
AGCACGACTTCTTCAAGTCCGCCATGCCCGAAGGCTACGTCCAGGAGCGCACCATCTTCTTC
AAGGACGACGGCAACTACAAGACCCGCGCCGAGGTGAAGTTCGAGGGCGACACCCTGGTG
AACCGCATCGAGCTGAAGGGCATCGACTTCAAGGAGGACGGCAACATCCTGGGGCACAAGC
TGGAGTACAACTACAACAGCCACAACGTCTATATCATGGCCGACAAGCAGAAGAACGGCATC
AAGGTGAACTTCAAGATCCGCCACAACATCGAGGACGGCAGCGTGCAGCTCGCCGACCACT
ACCAGCAGAACACCCCCATCGGCGACGGCCCCGTGCTGCTGCCCGACAACCACTACCTGA
GCACCCAGTCCGCCCTGAGCAAAGACCCCAACGAGAAGCGCGATCACATGGTCCTGCTGGA
GTTCGTGACCGCCGCCGGGATCACTCTCGGCATGGACGAGCTGTACAAG
[SEQ ID NO: 8] Linker
   TCCGGACTCAGATCTCGA
[SEQ ID NO: 9] α-Tubulin gene (Homo sapiens) 
GTGCGTGAGTGCATCTCCATCCACGTTGGCCAGGCTGGTGTCCAGATTGGCAATGCCTGCT
GGGAGCTCTACTGCCTGGAACACGGCATCCAGCCCGATGGCCAGATGCCAAGTGACAAGAC
CATTGGGGGAGGAGATGACTCCTTCAACACCTTCTTCAGTGAGACGGGCGCTGGCAAGCAC
GTGCCCCGGGCTGTGTTTGTAGACTTGGAACCCACAGTCATTGATGAAGTTCGCACTGGCAC
CTACCGCCAGCTCTTCCACCCTGAGCAGCTCATCACAGGCAAGGAAGATGCTGCCAATAACT
ATGCCCGAGGGCACTACACCATTGGCAAGGAGATCATTGACCTTGTGTTGGACCGAATTCGC
AAGCTGGCTGACCAGTGCACCGGTCTTCAGGGCTTCTTGGTTTTCCACAGCTTTGGTGGGG
GAACTGGTTCTGGGTTCACCTCCCTGCTCATGGAACGTCTCTCAGTTGATTATGGCAAGAAG
TCCAAGCTGGAGTTCTCCATTTACCCAGCACCCCAGGTTTCCACAGCTGTAGTTGAGCCCTA
CAACTCCATCCTCACCACCCACACCACCCTGGAGCACTCTGATTGTGCCTTCATGGTAGACA
ATGAGGCCATCTATGACATCTGTCGTAGAAACCTCGATATCGAGCGCCCAACCTACACTAACC
TTAACCGCCTTATTAGCCAGATTGTGTCCTCCATCACTGCTTCCCTGAGATTTGATGGAGCCC
TGAATGTTGACCTGACAGAATTCCAGACCAACCTGGTGCCCTACCCCCGCATCCACTTCCCT
CTGGCCACATATGCCCCTGTCATCTCTGCTGAGAAAGCCTACCATGAACAGCTTTCTGTAGCA
GAGATCACCAATGCTTGCTTTGAGCCAGCCAACCAGATGGTGAAATGTGACCCTCGCCATGG
TAAATACATGGCTTGCTGCCTGTTGTACCGTGGTGACGTGGTTCCCAAAGATGTCAATGCTGC
CATTGCCACCATCAAAACCAAGCGCAGCATCCAGTTTGTGGATTGGTGCCCCACTGGCTTCA
AGGTTGGCATCAACTACCAGCCTCCCACTGTGGTGCCTGGTGGAGACCTGGCCAAGGTACA
GAGAGCTGTGTGCATGCTGAGCAACACCACAGCCATTGCTGAGGCCTGGGCTCGCCTGGAC
CACAAGTTTGACCTGATGTATGCCAAGCGTGCCTTTGTTCACTGGTACGTGGGTGAGGGGAT
GGAGGAAGGCGAGTTTTCAGAGGCCCGTGAAGATATGGCTGCCCTTGAGAAGGATTATGAG
GAGGTTGGTGTGGATTCTGTTGAAGGAGAGGGTGAGGAAGAAGGAGAGGAATACTAA
[SEQ ID NO: 10] DHFR gene (Escherichia coli) 
ATGATCAGTCTGATTGCGGCGTTAGCGGTAGATCGCGTTATCGGCATGGAAAACGCCATGCC
GTGGAACCTGCCTGCCGATCTCGCCTGGTTTAAACGCAACACCTTAAATAAACCCGTGATTAT
GGGCCGCCATACCTGGGAATCAATCGGTCGTCCGTTGCCAGGACGCAAAAATATTATCCTCA
GCAGTCAACCGGGTACGGACGATCGCGTAACGTGGGTGAAGTCGGTGGATGAAGCCATCGC
GGCGTGTGGTGACGTACCAGAAATCATGGTGATTGGCGGCGGTCGCGTTTATGAACAGTTCT
TGCCAAAAGCGCAAAAACTGTATCTGACGCATATCGACGCAGAAGTGGAAGGCGACACCCAT
TTCCCGGATTACGAGCCGGATGACTGGGAATCGGTATTCAGCGAATTCCACGATGCTGATGC
 GCAGAACTCTCACAGCTATTGCTTTGAGATTCTGGAGCGGCGGTAA
[SEQ ID NO: 11] Primer DHFR-His_Fw
   GAAATTAATACGACTC
[SEQ ID NO: 12] Primer DHFR-His_Rv
   ATCCATTTAATTAGTGGTGATGGTGATGATGTCCACCGACTTCACCCACG
[SEQ ID NO: 13] DHFR protein
[SEQ ID NO: 14] DHFR-His protein 
MISLIAALAVDRVIGMENAMPWNLPADLAWFKRNTLNKPVIMGRPYLGINRSSVARTQKYYPQQS
TGYGRSRNVGEVGGHHHHHH
[SEQ ID NO: 15] Stem portion 1
   CCGCAUUA
[SEQ ID NO: 16] Stem portion 2
   UAGUGUGG
[SEQ ID NO: 17] Stem-loop structure 1
   CCGCAUUACCCCCCCCCCCUAGUGUGG
[SEQ ID NO: 18] Stem-loop structure 2
   GCGCAUUACCCCCCCCCCCUAGUGUGC

### Industrial Applicability

The single-stranded nucleic acid molecule or the composition according to one embodiment of the present invention can be used to prevent and/or treat genetic diseases, which are caused by genetic abnormalities, by normalizing gene expressions through inducing -1 frameshift.

### Cross-reference of related applications

The present application claims the benefit of priority to Japanese Patent Application No. 2020-041637, filed on March 11, 2020, the disclosure of which is incorporated herein by reference in its entirety.

## Claims

1. A single-stranded nucleic acid molecule, comprising in the direction from 5' end side to 3' end side a first sequence complementary to a target sequence of a gene of interest, and a second sequence forming a stem-loop structure.

2. The single-stranded nucleic acid molecule according to claim 1, wherein the stem-loop structure has a stem portion the ends of which are composed of cytosine and guanine.

3. The single-stranded nucleic acid molecule according to any one of claims 1 to 2, wherein the stem-loop structure has a loop portion that is primarily composed of cytosine.

4. The single-stranded nucleic acid molecule according to any one of claims 1 to 3, wherein the stem-loop structure has a stem portion composed of 4 base pairs to 20 base pairs.

5. The single-stranded nucleic acid molecule according to any one of claims 1 to 4, wherein the second sequence is represented in SEQ ID NO: 17 or SEQ ID NO:18.

6. The single-stranded nucleic acid molecule according to any one of claims 1 to 5, wherein the first sequence has 8 nucleotides to 16 nucleotides in length.

7. The single-stranded nucleic acid molecule according to any one of claims 1 to 6, wherein the gene has two or more consecutive identical nucleotides in 9 nucleotides to 15 nucleotides located upstream of the target sequence.

8. The single-stranded nucleic acid molecule according to any one of claims 1 to 6, wherein the gene has two or more sets of a portion composed of two or more consecutive identical nucleotides in 9 nucleotides to 15 nucleotides located upstream of the target sequence.

9. A composition for inducing -1 frameshift, comprising the single-stranded nucleic acid molecule according to any one of claims 1 to 8 as an active ingredient.

10. The composition according to claim 9, wherein the composition is a pharmaceutical composition for preventing and/or treating a genetic disease caused by impaired expression of a gene, through inducing -1 frameshift to normalize the expression of the gene.

11. The composition according to claim 10, wherein the genetic disease is selected from a group consisting of muscular dystrophy, cancer, multiple sclerosis, spinal muscle atrophy, Rett syndrome, amyotrophic lateral sclerosis, fragile X syndrome, Prader-Willi syndrome, xeroderma pigmentosum, porphyria, Werner syndrome, fibrodysplasia ossificans progressiva, infantile neuronal ceroid lipofuscinosis, Alzheimer's disease, Tay-Sachs disease, degenerative neurological disorder, Parkinson's disease, rheumatoid arthritis, graft-versus-host disease, arthritis, hematopoietic disease, von Willebrand disease, ataxia-telangiectasia, thalassemia, kidney stone, osteogenesis imperfecta, liver cirrhosis, neurofibromatosis, bullous disease, lysosomal storage disease, Hurler syndrome, cerebellar ataxia, tuberous sclerosis complex, familiar polycythemia, immunodeficiency, kidney disease, lung disease, cystic fibrosis, familial hypercholesterolemia, pigmentary retinopathy, amyloidosis, atherosclerosis, giantism, dwarfism, hypothyroidism, hyperthyroidism, aging, obesity, diabetes, Niemann-Pick disease and Marfan syndrome.

12. A method for treating and/or preventing a genetic disease, comprising administrating an effective amount of the single-stranded nucleic acid molecule according to any one of claims 1 to 8 or the composition according to claim 9, to a living individual developing or being at risk of developing the genetic disease caused by impaired expression of a gene of interest.

13. A method for ameliorating impaired expression of a gene, comprising administrating an effective amount of the single-stranded nucleic acid molecule according to any one of claims 1 to 8 or the composition according to claim 9, to a cell, a tissue, an organ or a living individual in which the impaired expression of the gene of interest is observed.

14. The method according to claim 12 or 13, the genetic disease is selected from a group consisting of muscular dystrophy, cancer, multiple sclerosis, spinal muscle atrophy, Rett syndrome, amyotrophic lateral sclerosis, fragile X syndrome, Prader-Willi syndrome, xeroderma pigmentosum, porphyria, Werner syndrome, fibrodysplasia ossificans progressiva, infantile neuronal ceroid lipofuscinosis, Alzheimer's disease, Tay-Sachs disease, degenerative neurological disorder, Parkinson's disease, rheumatoid arthritis, graft-versus-host disease, arthritis, hematopoietic disease, von Willebrand disease, ataxia-telangiectasia, thalassemia, kidney stone, osteogenesis imperfecta, liver cirrhosis, neurofibromatosis, bullous disease, lysosomal storage disease, Hurler syndrome, cerebellar ataxia, tuberous sclerosis complex, familiar polycythemia, immunodeficiency, kidney disease, lung disease, cystic fibrosis, familial hypercholesterolemia, pigmentary retinopathy, amyloidosis, atherosclerosis, giantism, dwarfism, hypothyroidism, hyperthyroidism, aging, obesity, diabetes, Niemann-Pick disease and Marfan syndrome.
